# EUROPEAN PATENT APPLICATION

(11) **EP 3 272 739 A1**
(43) Date of publication of application: **24.01.2018**
(21) Application number: 16460046.2
(22) Date of filing: 20.07.2016
(51) Int. Cl.: C07D 231/56, C07D 471/04, C07D 207/416, C07D 209/08, C07D 207/323, C07D 207/42, C07D 207/32, C07D 207/333, A61P 29/00, A61K 31/4035, A61K 31/40, A61K 31/403, A61K 31/437, A61K 31/416, A61P 25/14, A61P 37/00, A61P 35/00, C07D 209/44, C07D 209/52

(54) **SULFONYL UREA DERIVATIVES AND THEIR USE IN THE CONTROL OF INTERLEUKIN-1 ACTIVITY**

(71) Applicant: NodThera Limited, Edinburgh EH2 4DF (GB)
(72) Inventor: Levenets, Oleksandr, 30-392 Krakow (PL); Galezowski, Michal, 30-394 Kraków (PL); Bugaj, Marta, 32-447 Siepraw (PL); Woyciechowski, Jakub, 32-082 Bolechowice (PL)
(74) Representative: Stratagem IPM Limited

(57) **Abstract**

The present invention relates to compounds of Formula (I): 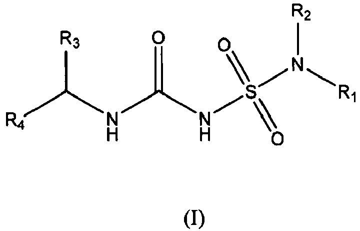 and to their pharmaceutically acceptable salts, pharmaceutical compositions, methods of use, and methods for their preparation. The compounds disclosed herein inhibit the maturation of cytokines of the IL-1 family by inhibiting inflammasomes and may be used in the treatment of disorders in which inflammasome activity is implicated, such as *inter alia* autoinflammatory and autoimmune diseases.

## Description

### FIELD OF THE INVENTION

The present invention concerns particular novel sulfonylurea compounds, or pharmaceutically-acceptable salt(s) thereof, which possess inflammasome inhibitory activity and are accordingly useful in methods of treatment of the human or animal body. The present invention also relates to processes for the preparation of these compounds, to pharmaceutical compositions comprising them, and to their use in the treatment of disorders in which inflammasome activity is implicated, such as autoinflammatory and autoimmune diseases.

### BACKGROUND OF THE INVENTION

Autoimmune diseases are associated with the overproduction of pro-inflammatory factors. One of them is interleukin-1 (IL-1), produced by activated macrophages, monocytes, fibroblasts, and other components of the innate immune system like dendritic cells, involved in a variety of cellular activities, including cell proliferation, differentiation and apoptosis (Seth L. al. Rev. Immunol. 2009. 27:621-68). Cytokines from the IL-1 family are highly active and, as important mediators of inflammation, primarily associated with acute and chronic inflammation (Sims J. et al. Nature Reviews Immunology 10, 89-102 (February 2010)). The overproduction of IL-1 is considered to be one of the major causes or mediators of most autoimmune and autoinflammatory diseases. Autoinflammatory diseases are characterized by recurrent and unprovoked inflammation in the absence of autoantibodies, infection, or antigen-specific T lymphocytes. Therefore, mostly the innate part of the immune system is involved in the disease pathogenesis. The mechanism of many inflammatory diseases appears to be due to increased secretion of IL-1β and treatment is usually based on reducing the activity of IL-1β.

On the other hand, autoimmune disorders are also mediated by the specific immune response with the involvement of T-cells and B-cells. Thus, both the innate and adaptive components of the immune system act together and inflammatory cytokines including IL-1 and IL18 are mediators of the origin of autoimmune diseases (Seth L. al. Rev. Immunol. 2009. 27:621-68) (Seth L. al. Rev. Immunol. 2009. 27:621-68).

The non-specific, innate immune response is the first line of defense against pathogenic organisms. Once activated, it regulates the inflammatory response by secreting pro-inflammatory cytokines, such as IL-1-type cytokines, in particular IL-1β. Due to a strong pro-inflammatory activity, the production of these cytokines and their activities are very tightly controlled. Under normal conditions, IL-1 and IL-18 are expressed at a very low level but induced at both transcriptional and translational levels upon stimulation of a wide range of pathogen associated molecular patterns (PAMPs) or danger associated molecular patterns (DAMPs). In addition, the activity of these cytokines is regulated at the level of posttranslational modification and secretion (Weber A. et al. Sci Signal. 2010 Jan 19;3(105)). Following the release of the active forms, their action is mediated by the binding to specific cellular receptor and activation of the signaling pathway leading to changes in gene expression. IL-1 mediates the expression of various genes involved in secondary inflammation that coordinates all aspects of local inflammation and also attract and activate cells of the adaptive immune system at the sites of infection.

IL-1β is expressed as an inactive precursor protein (the proform of IL-1β) that is located in the cell cytoplasm, not secreted and unable to bind and activate IL-1 receptors on target cells. To gain its biological activity, the proform of IL-1β needs to be processed by a specific protease, namely interleukin 1 convertase (ICE) or caspase-1. Caspase-1 recognizes a specific sequence of the proform and cleaves off a protein fragment. The shorter form of IL-1β gains the desired biological activity and is transported outside the cell where it gets into the bloodstream (Weber A, et al. Sci Signal. 2010 Jan 19;3(105)). IL-1α is processed by a different protease. Caspases are intracellular proteases that specifically recognize and cleave substrates at aspartic acid residues and their activities are very tightly controlled. In the unstimulated situation, caspase-1 is present in cells in an inactive state. The NLR family of proteins (Nucleotide binding domain (NOD) - like receptor) form central, cytoplasmatic molecular platforms, named inflammasomes (NALPs/IPAF) and NOD signalosomes_{,} capable of recognizing common pathogen molecules collectively referred to as pathogen-associated molecular patterns (PAMPs) - LPS, flagellin, lipoproteins, DNA, and RNA etc. (Beckley K. et al. Annu. Rev. Immunol. 2011. 29:707-35). Several types of inflammasomes have been described so far (NALP 1-14, IPAF, NAIP 1-6). Upon exposure to specific PAMPs / DAMPs, respective inflammasomes oligomerize and form a multiprotein complex, together with the adaptor protein ASC and caspase-1, leading to the photolytic cleavage of caspase-1 and subsequent cleavage of pro-IL-1β/IL-18 into its active and secreted forms (Franchi L. et al. Nat Immunol. 2009 March; 10(3): 241). The activation of inflammasome complexes is a necessary step for the formation of the active forms of IL-1β and IL-18 (Martinon F. et al. Annu. Rev. Immunol. 2009. 27:229-65). The relevance of the inflammasome complex as a potential target was confirmed at different levels. Studies with knockdown mice confirmed that the inflammasome components are crucial for the production of pro-IL-1β/IL-18 and mutations in NALP3 are found in a number of inflammatory human diseases that are characterized by increased systemic levels of IL-1β (Dinarello CA. Immunity. 2004 Mar;20(3):243-4). Moreover, several reports describing the development of small molecule inhibitors of in particular the NALP3 inflammasome have been published, further validating the inflammasome as a promising therapeutic target (Juliana C. et al. The Journal of Biological Chemistry vol. 285, no. 13, pp. 9792-9802, March 26, 2010; Coll RC. et al. Nat Med. 2015 Mar;21(3):248-55).

Surprisingly, applicants have found that the particular compounds of the present invention possess potent inhibitory activity against inflammasomes, including the NLRP3 inflammasome, and as such are expected to be useful in the treatment of diseases in which inflammasome activity is implicated, such as autoinflammatory and autoimmune diseases.

### SUMMARY OF THE INVENTION

According to a first aspect, the present invention relates to a compound of Formula (I): 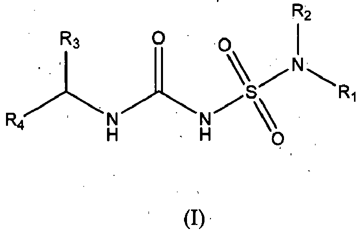 in which:
R₁ and R₂, together with the nitrogen atom to which they are attached to, form a 5 membered monocyclic heteroaryl ring system, wherein the monocyclic heteroaryl ring system, in addition to the nitrogen atom to which R₁ and R₂ are attached to, optionally comprises 1, 2 or 3 further heteroatoms independently selected from oxygen, nitrogen and sulfur; or
R₁ and R₂, together with the nitrogen atom to which they are attached to, form a 8, 9 or 10 membered bicyclic heteroaryl ring system, wherein the bicyclic heteroaryl ring system, in addition to the nitrogen atom to which R₁ and R₂ are attached to, optionally comprises 1, 2 or 3 further heteroatoms independently selected from oxygen, nitrogen and sulfur;
wherein said monocyclic heteroaryl ring system or said bicyclic heteroaryl ring system is optionally substituted by 1, 2, or 3 substituents independently selected from (1-6C)alkyl, (2-6C)alkenylene, (2-6C)alkynylene, (3-8C)cycloalkyl, halo, nitro, cyano, CF₃, oxo, OR₅, N(R₆)(R₇), NR₅C(O)R₆, NR₆S(O)₂R₈, N(R₅)C(O)N(R₆)(R₇), S(O)₂R₈, S(O)R₈, S(O)(NR₅)(R₈), S(O)₂N(R₆)(R₇), C(O)OR₅, C(O)N(R₆)(R₇), C(O)R₈, C(NOR₅)(R₈), OC(O)N(R₆)(R₇), OC(O)R₈, phenyl, a 5 or 6 membered monocyclic heteroaryl ring system comprising 1, 2 or 3 heteroatoms independently selected from oxygen, nitrogen and sulfur, and a 3, 4, 5, or 6 membered monocyclic heterocyclyl ring system comprising 1 or 2 heteroatoms independently selected from oxygen, nitrogen and sulfur, wherein said (1-6C)alkyl, (2-6C)alkenylene, (2-6C)alkynylene, (3-8C)cycloalkyl, phenyl, 5 or 6 membered monocyclic heteroaryl ring system or 3, 4, 5, or 6 membered monocyclic heterocyclyl ring system is optionally substituted by 1, 2, 3 or 4 substituents independently selected from halo, cyano, oxo, CF₃, OR₅, N(R₆)(R₇), NR₅C(O)R₆, SR₅, C(O)N(R₆)(R₇), S(O)₂R₈, SOR₈, S(O)₂N(R₅)(R₆) and NR₅S(O)₂R₆;
R₃ and R₄, together with the carbon atom to which they are attached to, form a 12, 13, 14, 15 or 16 membered tricyclic partially unsaturated heterocyclic or carbocyclic ring system, wherein said tricyclic ring system is optionally substituted by 1, 2, 3 or 4 substituents independently selected from (1-6C)alkyl, (2-6C)alkenylene, (2-6C)alkynylene, (3-8C)cycloalkyl, (1-3C)alkoxy, halo, oxo., hydroxy, cyano, amino, (1-3C)alkylamino, di-[(1-3C)alkyl]-amino, CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃;
R₅, R₆ arid R₇ are each independently selected from H, (1-6C)alkyl, CF₃, phenyl, a 5 or 6 membered monocyclic heteroaryl ring system comprising 1, 2 or 3 heteroatoms independently selected from oxygen, nitrogen and sulfur, a 3, 4, 5 or 6 membered monocyclic heterocyclyl ring system comprising 1 or 2 heteroatoms independently selected from oxygen, nitrogen and sulfur and a 3, 4, 5 or 6 membered saturated or partially unsaturated carbocyclic ring system, wherein said (1-6C)alkyl, phenyl, monocyclic heteroaryl ring, monocyclic heterocyclyl ring or carbocyclic ring system is optionally substituted with 1 or 2 substituents independently selected from halo, amino, methylamino, di(methyl)amino, nitro, hydroxy, methoxy, oxo, cyano, C(O)NH₂, C(O)NHCH₃, C(O)N(CH₃)(CH₃), CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃;
each R₈ is independently selected from (1-6C)alkyl, CF₃, phenyl, a 5 or 6 membered monocyclic heteroaryl ring system comprising 1, 2 or 3 heteroatoms independently selected from oxygen, nitrogen and sulfur, a 3, 4, 5 or 6 membered monocyclic heterocyclyl ring system comprising 1 or 2 heteroatoms independently selected from oxygen, nitrogen and sulfur and a 3, 4, 5 or 6 membered saturated or partially unsaturated carbocyclic ring system, wherein said (1-6C)alkyl, phenyl, monocyclic heteroaryl ring, monocyclic heterocyclyl ring or carbocyclic ring system is optionally substituted with 1 or 2 substituents independently selected from halo, amino, methylamino, di(methyl)amino, nitro, hydroxy, methoxy, oxo, cyano, C(O)NH₂, C(O)NHCH₃, C(O)N(CH₃)(CH₃), CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃; or a pharmaceutically acceptable salt thereof.

According to a further aspect of the present invention, there is provided a pharmaceutical composition comprising a compound as defined herein, or a pharmaceutically acceptable salt thereof, in admixture with a pharmaceutically acceptable diluent or carrier.

According to a further aspect of the present invention, there is provided a method of inhibiting inflammasome (such as the NLRP3 inflammasome) activity *in vitro* or *in vivo,* said method comprising contacting a cell with an effective amount of a compound of Formula (I) or a pharmaceutically acceptable salt thereof as defined herein.

According to a further aspect of the present invention, there is provided a method of treating a disease or disorder in which inflammasome activity is implicated in a patient in need of such treatment, said method comprising administering to said patient a therapeutically effective amount of a compound of Formula (I) or a pharmaceutically acceptable salt thereof as defined herein, or a pharmaceutical composition as defined herein.

Inflammasome activity is implicated in a wide range of disorders and a compound of Formula I may therefore be useful as a therapeutic agent for a number of indications including, but not limited to, cryopyrin-associated autoinflammatory syndromes (CAPS) including familial cold autoinflammatory syndrome (FCAS), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), gout, rheumatoid arthritis, Crohn's disease, COPD, fibrosis, obesity, type 2 diabetes, multiple sclerosis and neuro-inflammation occurring in protein miss-folding diseases (such as Prion diseases), Parkinson's disease, Alzheimer's disease, cancers (such as melanoma, sarcoma, colorectal cancer, myeloma mesothelioma, lung cancer and breast cancer), meningitis, salpingitis, septic shock, disseminated intravascular coagulation, adult respiratory distress syndrome, acute or chronic inflammation, cholangitis, colitis, encephalitis, endocarditis, glomerulonephritis, hepatitis, myocarditis, pancreatitis, pericarditis, reperfusion injury, vasculitis, acute and delayed hypersensitivity, graft rejection, and graft-versus-host disease, periodonate diseases, interstitial pulmonary fibrosis, cirrhosis, systemic sclerosis, keloid formation, cachexia, percussion injury, depression, atherosclerosis (including cardiomyopathy, myocarditis and heart failure), osteoporosis and Adult and Juvenile Onset Still's Disease.

According to a further aspect of the present invention, there is provided a method of treating an autoinflammatory disorder, an autoimmune disorder, a neurodegenerative disease or cancer in a patient in need of such treatment, said method comprising administering to said patient a therapeutically effective amount of a compound of Formula (I) or a pharmaceutically acceptable salt thereof as defined herein, or a pharmaceutical composition as defined herein.

According to a further aspect of the present invention, there is provided a method of treating a disorder selected from cryopyrin-associated autoinflammatory syndromes (CAPS) including familial cold autoinflammatory syndrome (FCAS), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), gout, rheumatoid arthritis, Crohn's disease, COPD, fibrosis, obesity, type 2 diabetes, multiple sclerosis and neuro-inflammation occurring in protein miss-folding diseases (such as Prion diseases), Parkinson's disease, Alzheimer's disease, cancers (such as melanoma, sarcoma, colorectal cancer, myeloma mesothelioma, lung cancer and breast cancer), meningitis, salpingitis, septic shock, disseminated intravascular coagulation, adult respiratory distress syndrome, acute or chronic inflammation, cholangitis, colitis, encephalitis, endocarditis, glomerulonephritis, hepatitis, myocarditis, pancreatitis, pericarditis, reperfusion injury, vasculitis, acute and delayed hypersensitivity, graft rejection, and graft-versus-host disease, periodonate diseases, interstitial pulmonary fibrosis, cirrhosis, systemic sclerosis, keloid formation, cachexia, percussion injury, depression, atherosclerosis (including cardiomyopathy, myocarditis and heart failure), osteoporosis and Adult and Juvenile Onset Still's Disease in a patient in need of such treatment, said method comprising administering to said patient a therapeutically effective amount of a compound of Formula (I) or a pharmaceutically acceptable salt thereof as defined herein, or a pharmaceutical composition as defined herein.

According to a further aspect of the present invention, there is provided a method of treating an autoinflammatory disorder and/or an autoimmune disorder selected from cryopyrin-associated autoinflammatory syndromes (CAPS) including familial cold autoinflammatory syndrome (FCAS), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), gout, rheumatoid arthritis, Crohn's disease, COPD, fibrosis, obesity, type 2 diabetes, multiple sclerosis and neuro-inflammation occurring in protein miss-folding diseases, such as Prion diseases in a patient in need of such treatment, said method comprising administering to said patient a therapeutically effective amount of a compound of Formula (I) or a pharmaceutically acceptable salt thereof as defined herein, or a pharmaceutical composition as defined herein.

According to a further aspect of the present invention, there is provided a method of treating a neurodegenerative disease such as Parkinson's disease or Alzheimer's disease in a patient in need of such treatment, said method comprising administering to said patient a therapeutically effective amount of a compound of Formula (I) or a pharmaceutically acceptable salt thereof as defined herein, or a pharmaceutical composition as defined herein.

According to a further aspect of the present invention, there is provided a compound of Formula (I), or a pharmaceutically acceptable salt thereof, or a pharmaceutical composition as defined herein for use in therapy.

According to a further aspect of the present invention, there is provided a compound of Formula (I) or a pharmaceutically acceptable salt thereof as defined herein, or a pharmaceutical composition as defined herein, for use in the treatment of a disorder in which inflammasome activity is implicated.

According to a further aspect of the present invention, there is provided a compound of Formula (I), or a pharmaceutically acceptable salt thereof, or a pharmaceutical composition as defined herein for use in the treatment of a disorder selected from cryopyrin-associated autoinflammatory syndromes (CAPS) including familial cold autoinflammatory syndrome (FCAS), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), gout, rheumatoid arthritis, Crohn's disease, COPD, fibrosis, obesity, type 2 diabetes, multiple sclerosis and neuro-inflammation occurring in protein miss-folding diseases (such as Prion diseases), Parkinson's disease, Alzheimer's disease, cancers (such as melanoma, sarcoma, colorectal cancer, myeloma mesothelioma, lung cancer and breast cancer), meningitis, salpingitis, septic shock, disseminated intravascular coagulation, adult respiratory distress syndrome, acute or chronic inflammation, cholangitis, colitis, encephalitis, endocarditis, glomerulonephritis, hepatitis, myocarditis, pancreatitis, pericarditis, reperfusion injury, vasculitis, acute and delayed hypersensitivity, graft rejection, and graft-versus-host disease, periodonate diseases, interstitial pulmonary fibrosis, cirrhosis, systemic sclerosis, keloid formation, cachexia, percussion injury, depression, atherosclerosis (including cardiomyopathy, myocarditis and heart failure), osteoporosis or Adult and Juvenile Onset Still's Disease.

According to a further aspect of the present invention, there is provided a compound of Formula (I), or a pharmaceutically acceptable salt thereof, or a pharmaceutical composition as defined herein for use in the treatment of an autoinflammatory disorder, an autoimmune disorder, a neurodegenerative disease or cancer. In a particular embodiment, the autoinflammatory or autoimmune disorder is a cryopyrin-associated autoinflammatory syndrome (CAPS) such as for example familial cold autoinflammatory syndrome (FCAS), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), gout, rheumatoid arthritis, Crohn's disease, COPD, fibrosis, obesity, type 2 diabetes, multiple sclerosis or neuro-inflammation occurring in protein miss-folding diseases, such as Prion diseases. In a further embodiment, the neurodegenerative disease is Parkinson's disease or Alzheimer's disease.

According to a further aspect of the present invention, there is provided the use of a compound of Formula (I), or a pharmaceutically acceptable salt thereof, as defined herein in the manufacture of a medicament for the treatment of a disorder, in which inflammasome activity is implicated.

According to a particular aspect of the present invention, there is provided the use of a compound of Formula (I), or a pharmaceutically acceptable salt thereof, as defined herein in the manufacture of a medicament for the treatment of an autoinflammatory disorder, an autoimmune disorder, a neurodegenerative disease or cancer. Suitably, the autoinflammatory or autoimmune disorder is cryopyrin-associated autoinflammatory syndrome (CAPS) such as for example familial cold autoinflammatory syndrome (FCAS), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), gout, rheumatoid arthritis, Crohn's disease, COPD, fibrosis, obesity, type 2 diabetes, multiple sclerosis or neuro-inflammation occurring in protein miss-folding diseases, such as Prion diseases. Suitably, the neurodegenerative disease is Parkinson's disease or Alzheimer's disease.

According to a further aspect of the present invention, there is provided a process for preparing a compound of Formula (I), or a pharmaceutically acceptable salt thereof, as defined herein.

According to a further aspect of the present invention, there is provided a compound of Formula (I), or a pharmaceutically acceptable salt thereof, obtainable by, or obtained by, or directly obtained by a process of preparing a compound as defined herein.

According to a further aspect of the present invention, there are provided novel intermediates as defined herein which are suitable for use in any one of the synthetic methods set out herein.

### DETAILED DESCRIPTION OF THE INVENTION

### DEFINITIONS

Unless otherwise stated, the following terms used in the specification and claims have the following meanings set out below.

It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms.

A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

In this specification the term "alkyl" includes both straight and branched chain alkyl groups such as propyl, isopropyl and *t*-butyl. However, references to individual alkyl groups such as "propyl" are specific for the straight chain version only and references to individual branched chain alkyl groups such as "isopropyl" are specific for the branched chain version only. For example, "(1-6C)alkyl" includes (1-4C)alkyl, (1-3C)alkyl, propyl, isopropyl and *t*-butyl. A similar convention applies to other radicals, for example "(1-6C)alkoxy".

An "alkylene," "alkenylene," or "alkynylene" group is an alkyl, alkenyl, or alkynyl group that is positioned between and serves to connect two other chemical groups. Thus, "(1-6C)alkylene" means a linear saturated divalent hydrocarbon radical of one to six carbon atoms or a branched saturated divalent hydrocarbon radical of three to six carbon atoms, for example, methylene, ethylene, propylene, 2-methylpropylene, pentylene, and the like.

"(2-6C)alkenylene" means a linear divalent hydrocarbon radical of two to six carbon atoms or a branched divalent hydrocarbon radical of three to six carbon atoms, containing at least one double bond, for example, as in ethenylene, 2,4-pentadienylene, and the like.

"(2-6C)alkynylene" means a linear divalent hydrocarbon radical of two to six carbon atoms or a branched divalent hydrocarbon radical of three to six carbon atoms, containing at least one triple bond, for example, as in ethynylene, propynylene, and butynylene and the like.

"(3-8C)cycloalkyl" means a hydrocarbon ring containing from 3 to 8 carbon atoms, for example, cyclopropyl, cyclobutyl, cyclopentyl, cyclohexyl, cycloheptyl or bicyclo[2.2.1]heptyl.

The term "halo" refers to fluoro, chloro, bromo and iodo.

Suitable values for the term "(1-6C)alkoxy) include methoxy, ethoxy, propoxy, isopropoxy and butoxy.

Suitable values for the term "(1-3C)alkylamino" include methylamino, ethylamino, propylamino, isopropylamino and butylamino.

Suitable values for the term "di-[(1-3C)alkyl]-amino" include dimethylamino, diethylamino, *N*-ethyl-*N*-methylamino and diisopropylamino.

The term "aryl" means a cyclic or polycyclic aromatic ring having from 5 to 12 carbon atoms. The term aryl includes both monovalent species and divalent species. Examples of aryl groups include, but are not limited to, phenyl, biphenyl, naphthyl and the like. Conveniently, an aryl is phenyl.

The term "5 membered monocyclic heteroaryl ring system" when used to define the ring system formed by R₁ and R₂, together with the nitrogen atom to which they are attached to, refers to a 5 membered aromatic ring system, wherein the ring system, in addition to the nitrogen atom to which R₁ and R₂ are attached to, optionally comprises 1, 2 or 3 further heteroatoms independently selected from oxygen, nitrogen and sulfur. Suitable examples include pyrrolyl, isoxazolyl, thiazolyl, isothiazolyl, pyrazolyl, imidazolyl, oxadiazolyl, thiadiazolyl, triazolyl and tetrazolyl. Conveniently, the 5 membered monocyclic heteroaryl ring system is pyrrolyl.

The term "8, 9 or 10 membered bicyclic heteroaryl ring system" when used to define the ring system formed by R₁ and R₂, together with the nitrogen atom to which they are attached to, refers to a 8, 9 or 10 membered aromatic ring system, wherein the ring system, in addition to the nitrogen atom to which R₁ and R₂ are attached to, optionally comprises 1, 2 or 3 heteroatoms independently selected from oxygen, nitrogen and sulfur. Suitable examples include indolyl, isoindolyl, indazolyl, benzimidazolyl, benzoxathiazolyl, benzoxadiazolyl, benzothiadiazolyl, quinolinyl, isoquinolinyl, purinyl, 1,8-naphthyridyl, pteridyl, 1H-pyrrolo[3,2-b]pyridinyl, 1H-pyrrolo[2,3-c]pyridinyl, pyrido[3,2-d]pyrimidyl and pyridoimidazolyl. The term "8, 9 or 10 membered bicyclic heteroaryl ring system" also covers partially aromatic bicyclic ring systems wherein the first ring (i.e. the ring that includes the nitrogen atom directly connected to the sulfonyl group of Formula (I)) is aromatic and the other second ring is non-aromatic, saturated or partially saturated. Suitable examples of partially aromatic bicyclic ring systems include for example, 4,5,6,7-tetrahydroindolyl, 4,5,6,7-tetrahydroisoindolyl and 2H,4H,5H,6H-cyclopenta[c]pyrrolyl.

The term "5 or 6 membered monocyclic heteroaryl ring system" refers to a 5 or 6 membered aromatic ring system comprising 1, 2 or 3 heteroatoms independently selected from oxygen, nitrogen and sulfur. Suitable examples include pyrrolyl, isoxazolyl, thiazolyl, isothiazolyl, pyrazolyl, imidazolyl, oxadiazolyl, thiadiazolyl, triazolyl, tetrazolyl, pyridyl, pyrimidinyl, pyrazinyl and pyridazinyl.

The term "3, 4, 5, or 6 membered monocyclic heterocyclyl ring system" refers to a 3, 4, 5, or 6 membered non-aromatic saturated or partially saturated heterocyclic ring system, wherein the ring system optionally comprises 1 or 2 heteroatoms independently selected from oxygen, nitrogen and sulfur, wherein a ring sulfur atom is optionally oxidized to form the S-oxide(s). Suitable examples include oxiranyl, aziridinyl, azetidinyl, oxetanyl, pyrrolinyl, pyrrolidinyl, imidazolinyl, imidazolidinyl, pyrazolinyl, pyrazolidinyl, morpholinyl, thiomorpholinyl, piperidinyl, homopiperidinyl, piperazinyl, homopiperazinyl, tetrahydrofuranyl, tetrahydropyran and tetrahydro-1,4-thiazinyl.

The term "12, 13, 14, 15 or 16 membered tricyclic partially unsaturated heterocyclic ring system" when used to define the ring system formed by R₃ and R₄, together with the carbon atom to which they are attached to, refers to a 12, 13, 14, 15 or 16 partially unsaturated heterocyclic ring system, which comprises 1 or 2 heteroatoms independently selected from oxygen, nitrogen and sulfur, wherein a ring sulfur atom is optionally oxidized to form the S-oxide(s). Suitable examples include rings such as 2-azatricyclo[7.3.0.0³,⁷]dodeca-1,3(7),8-trienyl, 1,2,3,4,5,6,7,8-octahydroacridinyl, 7-azatricyclo[7.3.0.0²,⁶]dodeca-1,6,8-trienyl, 1,2,3,4,7,8,9,10-octahydrophenanthridiriyl, 1 H,2H,3H,6H,7H,8H,9H-cyclopenta[c]isoquinolinyl, 1H,2H,3H,6H,7H_{,}8H,9H-cyclopenta[c]quinolonyl, 1H,2H,3H,5H,6H,7H,8H-cyclopenta[b]quinolonyl, 1H,2H,3H,5H,6H,7H-cyclopenta[b]pyrrolizinyl, 1H,2H,3H,5H,6H,7H,8H-cyclohexa[b]pyrrolizinyl, 1H,2H,3H,5H,6H,7H-cyclopenta[b]pyrrolizinyl and 1H,2H,3H,5H,6H,7H,8H-cyclopenta[b]indolizinyl. It will be understood by the person skilled in the art that when the carbon atom to which both R₃ and R₄ are attached is sp²-hybridized, the said carbon atom will have no hydrogen attached.

The term "12, 13, 14, 15 or 16 membered tricyclic partially unsaturated carbocyclic ring system" when used to define the ring system formed by R₃ and R₄, together with the carbon atom to which they are attached to, refers to a 12, 13, 14, 15 or 16 partially unsaturated carbocyclic ring system comprising only carbon atoms. Suitable examples include rings such as 1,2,3,5,6;7-hexahydro-s-indacenyl, 1H,2H,3H,6H,7H,8H,9H-cyclopenta[a]naphthalenyl, 1,2,3,6,7,8-hexahydroas-indacenyl, 1,2,3,4,5,6,7,8-octahydroanthracenyl, 1,2,3,4,5,6,7,8-octahydrophenanthrenyl and 1H,2H,3H,5H,6H,7H,8H-cyciopenta[b]naphthalenyl. In a particular group of compounds of the Formula I, suitable rings are 1,2,3,5,6,7-hexahydro-s-indacenyl, 1H,2H,3H,6H,7H,8H,9H-cyclopenta[a]naphthalenyl and 1,2,3,6,7,8-hexahydroas-indacenyl. Conveniently, the said ring is 1,2,3,5,6,7-hexahydro-s-indacenyl. It will be understood by the person skilled in the art that when the carbon atom to which both R₃ and R₄ are attached is sp²-hybridized, the said carbon atom will have no hydrogen attached.

The term "3, 4, 5 or 6 membered saturated or partially unsaturated carbocyclic ring system" refers to a monocyclic ring system comprising only carbon atoms. Suitable examples include cyclopropanyl, cyclopentanyl, cyclohexanyl and cyclohexenyl.

The phrase "compound of the invention" means those compounds which are disclosed herein, both generically and specifically.

### COMPOUNDS OF THE INVENTION

According to a first aspect, the present invention relates to a compound of Formula (I): 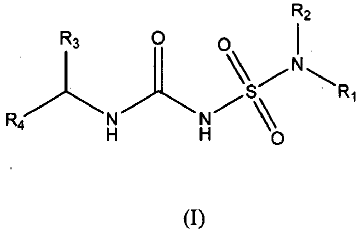 in which:
R₁ and R₂, together with the nitrogen atom to which they are attached to, form a 5 membered monocyclic heteroaryl ring system, wherein the monocyclic heteroaryl ring system, in addition to the nitrogen atom to which R₁ and R₂ are attached to, optionally comprises 1, 2 or 3 further heteroatoms independently selected from oxygen, nitrogen and sulfur; or
R₁ and R₂, together with the nitrogen atom to which they are attached to, form a 8, 9 or 10 membered bicyclic heteroaryl ring system, wherein the bicyclic heteroaryl ring system, in addition to the nitrogen atom to which R₁ and R₂ are attached to, optionally comprises 1, 2 or 3 further heteroatoms independently selected from oxygen, nitrogen and sulfur;
wherein said monocyclic heteroaryl ring system or said bicyclic heteroaryl ring system is optionally substituted by 1, 2, or 3 substituents independently selected from (1-6C)alkyl, (2-6C)alkenylene, (2-6C)alkynylene, (3-8C)cycloalkyl, halo, nitro, cyano, CF₃, oxo, OR₅, N(R₆)(R₇), NR₅C(O)R₆, NR₆S(O)₂R₈, N(R₅)C(O)N(R₆)(R₇), S(O)₂R₈, S(O)R₈, S(O)(NR₅)(R₈), S(O)₂N(R₆)(R₇), C(O)OR₅, C(O)N(R₆)(R₇), C(O)R₈, C(NOR₅)(R₈), OC(O)N(R₆)(R₇), OC(O)R₈, phenyl, a 5 or 6 membered monocyclic heteroaryl ring system comprising 1, 2 or 3 heteroatoms independently selected from oxygen, nitrogen and sulfur, and a 3, 4, 5, or 6 membered monocyclic heterocyclyl ring system comprising 1 or 2 heteroatoms independently selected from oxygen, nitrogen and sulfur, wherein said (1-6C)alkyl, (2-6C)alkenylene, (2-6C)alkynylene, (3-8C)cycloalkyl, phenyl, 5 or 6 membered monocyclic heteroaryl ring system or 3, 4, 5, or 6 membered monocyclic heterocyclyl ring system is optionally substituted by 1, 2, 3 or 4 substituents independently selected from halo, cyano, oxo, CF₃, OR₅, N(R₆)(R₇), NR₅C(O)R₆, SR₅, C(O)N(R₆)(R₇), S(O)₂R₈, SOR₈, S(O)₂N(R₅)(R₆) and NR₅S(O)₂R₆;
R₃ and R₄, together with the carbon atom to which they are attached to, form a 12, 13, 14, 15 or 16 membered tricyclic partially unsaturated heterocyclic or carbocyclic ring system, wherein said tricyclic ring system is optionally substituted by 1, 2, 3 or 4 substituents independently selected from (1-6C)alkyl, (2-6C)alkenylene, (2-6C)alkynylene, (3-8C)cycloalkyl, (1-3C)alkoxy, halo, oxo, hydroxy, cyano, amino, (1-3C)alkylamino, di-[(1-3C)alkyl]-amino, CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃;
R₅, R₆ and R₇ are each independently selected from H, (1-6C)alkyl, CF₃, phenyl, a 5 or 6 membered monocyclic heteroaryl ring system comprising 1, 2 or 3 heteroatoms independently selected from oxygen, nitrogen and sulfur, a 3, 4, 5 or 6 membered monocyclic heterocyclyl ring system comprising 1 or 2 heteroatoms independently selected from oxygen, nitrogen and sulfur and a 3, 4, 5 or 6 membered saturated or partially unsaturated carbocyclic ring system, wherein said (1-6C)alkyl, phenyl, monocyclic heteroaryl ring, monocyclic heterocyclyl ring or carbocyclic ring system is optionally substituted with 1 or 2 substituents independently selected from halo, amino, methylamino, di(methyl)amino, nitro, hydroxy, methoxy, oxo, cyano, C(O)NH₂, C(O)NHCH₃, C(O)N(CH₃)(CH₃), CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃;
each R₈ is independently selected from (1-6C)alkyl, CF₃, phenyl, a 5 or 6 membered monocyclic heteroaryl ring system comprising 1, 2 or 3 heteroatoms independently selected from oxygen, nitrogen and sulfur, a 3, 4, 5 or 6 membered monocyclic heterocyclyl ring system comprising 1 or 2 heteroatoms independently selected from oxygen, nitrogen and sulfur and a 3, 4, 5 or 6 membered saturated or partially unsaturated carbocyclic ring system, wherein said (1-6C)alkyl, phenyl, monocyclic heteroaryl ring, monocyclic heterocyclyl ring or carbocyclic ring system is optionally substituted with 1 or 2 substituents independently selected from halo, amino, methylamino, di(methyl)amino, nitro, hydroxy, methoxy, oxo, cyano, C(O)NH₂, C(O)NHCH₃, C(O)N(CH₃)(CH₃), CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃; or a pharmaceutically acceptable salt thereof.

For the avoidance of doubt it is to be understood that where in this specification a group is qualified by 'hereinbefore defined' or 'defined hereinbefore' the said group encompasses the first occurring and broadest definition as well as each and all of the particular definitions for that group.

Particular compounds of the invention include, for example, compounds of the Formula (I), or pharmaceutically acceptable salt thereof, wherein, unless otherwise stated, each of R₁, R₂, R₃, R₄ and any associated substituent groups has any of the meanings defined hereinbefore or in any of paragraphs (1) to (21) hereinafter:-
(1) R₁ and R₂, together with the nitrogen atom to which they are attached to, form a 5 membered monocyclic heteroaryl ring system selected from pyrrolyl, isoxazolyl, thiazolyl, isothiazolyl, pyrazolyl, imidazolyl, oxadiazolyl, thiadiazolyl, triazolyl and tetrazolyl; or R₁ and R₂, together with the nitrogen atom to which they are attached to, form a 8, 9 or 10 membered bicyclic heteroaryl ring system selected from indolyl, isoindolyl, indazolyl, 4,5,6,7-tetrahydroindolyl, 4,5,6,7-tetrahydroisoindolyl, benzimidazolyl, benzoxathiazolyl, benzoxadiazolyl, benzothiadiazolyl, quinolinyl, isoquinolinyl, purinyl, 1,8-naphthyridyl, pteridyl, 1H-pyrrolo[3,2-b]pyridinyl, 1H-pyrrolo[2,3-c]pyridinyl, 2H,4H,5H,6H-cyclopenta[c]pyrrolyl, pyrido[3,2-d]pyrimidyl and pyridoimidazolyl, wherein said monocyclic heteroaryl ring system or said bicyclic heteroaryl ring system is optionally substituted as defined in Formula (I);
(2) R₁ and R₂, together with the nitrogen atom to which they are attached to, form a 5 membered monocyclic heteroaryl ring system selected from pyrrolyl, isoxazolyl, thiazolyl, isothiazolyl, pyrazolyl, imidazolyl, oxadiazolyl, thiadiazolyl, triazolyl and tetrazolyl, wherein said monocyclic heteroaryl ring system is optionally substituted as defined in Formula (I);
(3) R₁ and R₂, together with the nitrogen atom to which they are attached to, form a 8, 9 or 10 membered bicyclic heteroaryl ring system selected from indolyl, isoindolyl, indazolyl, 4,5,6,7-tetrahydroindolyl, 4,5,6,7-tetrahydroisoindolyl, benzimidazolyl, benzoxathiazolyl, benzoxadiazolyl, benzothiadiazolyl, quinolinyl, isoquinolinyl, purinyl, 1,8-naphthyridyl, pteridyl, 1H-pyrrolo[3,2-b]pyridinyl, 1H-pyrrolo[2,3-c]pyridinyl, 2H,4H,5H,6H-cyclopenta[c]pyrrolyl, pyrido[3,2-d]pyrimidyl and pyridoimidazolyl, wherein said bicyclic heteroaryl ring system is optionally substituted as defined in Formula (I);
(4) R₁ and R₂, together with the nitrogen atom to which they are attached to, form a ring system selected from pyrrolyl, indolyl, indazolyl, 1H-pyrrolo[3,2-b]pyridinyl, 1H-pyrrolo[2,3-c]pyridinyl and 2H,4H,5H,6H-cyclopenta[c]pyrrolyl, wherein said monocyclic heteroaryl ring system or said bicyclic heteroaryl ring system is optionally substituted as defined in Formula (I);
(5) R₁ and R₂, together with the nitrogen atom to which they are attached to, form a ring system selected from pyrrolyl, 1H-indolyl, 1H-indazolyl, 4,5,6,7-tetrahydroisoindolyl, 1H-pyrrolo[3,2-b]pyridinyl, 1H-pyrrolo[2,3-c]pyridinyl and 2H,4H,5H,6H-cyclopenta[c]pyrrolyl, wherein said monocyclic heteroaryl ring system or said bicyclic heteroaryl ring system is optionally substituted as defined in Formula (I);
(6) R₁ and R₂, together with the nitrogen atom to which they are attached to, form a pyrrolyl ring, wherein said pyrrolyl ring system is optionally substituted as defined in Formula (I);
(7) R₁ and R₂, together with the nitrogen atom to which they are attached to, form a ring system selected from pyrrolyl, indolyl, indazolyl, 4,5,6,7-tetrahydroisoindolyl, 1H-pyrrolo[3,2-b]pyridinyl, 1H-pyrrolo[2,3-c]pyridinyl and 2H,4H,5H,6H-cyclopenta[c]pyrrolyl, wherein said ring system is optionally substituted by 1, 2, or 3 substituents independently selected from (1-6C)alkyl, (2-6C)alkenylene, (2-6C)alkynylene, (3-8C)cycloalkyl, halo, nitro, cyano, CF₃, oxo, OR₅, N(R₆)(R₇), NR₅C(O)R₆, NR₆S(O)₂R₈, N(R₅)C(O)N(R₆)(R₇), S(O)₂R₈, S(O)R₈, S(O)(NR₅)(R₈), S(O)₂N(R₆)(R₇), C(O)OR₅, C(O)N(-R₆)(R₇), C(O)R₈, C(NOR₅)(R₈), OC(O)N(R₆)(R₇), OC(O)R₈, phenyl, a 5 or 6 membered monocyclic heteroaryl ring system comprising 1 or 2 heteroatoms independently selected from oxygen, nitrogen and sulfur, and a 3, 4, 5, or 6 membered monocyclic heterocyclyl ring system comprising 1 or 2 heteroatoms independently selected from oxygen, nitrogen and sulfur, wherein said (1-6C)alkyl, (2-6C)alkenylene, (2-6C)alkynylene, (3-8C)cycloalkyl, phenyl, monocyclic heteroaryl ring system or monocyclic heterocyclyl ring system is optionally substituted by 1, 2, 3 or 4 substituents independently selected from halo, cyano, oxo, CF₃, OR₅, N(R₆)(R₇), NR₅C(O)R₆, SR₅, C(O)N(R₆)(R₇), S(O)₂R₈, SOR₈, S(O)₂N(R₅)(R₆) and NR₅S(O)₂R₆;
(8) R₁ and R₂, together with the nitrogen atom to which they are attached to, form a ring system selected from pyrrolyl, indolyl, indazolyl, 4,5,6,7-tetrahydroisoindolyl, 1H-pyrrolo[3;2-b]pyridinyl; 1H-pyrrolo[2,3-c]pyridinyl and 2H,4H,5H,6H-cyclopenta[c]pyrrolyl, wherein said ring system is optionally substituted by 1, 2, or 3 substituents independently selected from (1-6C)alkyl, (2-6C)alkenylene, (2-6C)alkynylene, (3-8C)cycloalkyl, halo, cyano, CF₃, oxo, amino, (1-3C)alkylamino, di-[(1-3C)alkyl]-amino, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃, OR₅, C(O)OR₅, C(O)R₈ and phenyl, wherein said (1-6C)alkyl, (2-6C)alkenylene, (2-6C)alkynylene, (3-8C)cycloalkyl or phenyl is optionally substituted by 1, 2, 3 or 4 substituents independently selected from halo, CF₃ and hydroxy, wherein each R₈ is independently selected from (1-6C)alkyl, (1-3C)alkoxy and CF₃, and wherein each R₅ is (1-6C)alkyl;
(9) R₁ and R₂, together with the nitrogen atom to which they are attached to, form a ring system selected from pyrrolyl, indolyl, indazolyl, 4,5,6,7-tetrahydroisoindolyl, 1H-pyrrolo[3,2-b]pyridinyl, 1H-pyrrolo[2,3-c]pyridinyl and 2H,4H,5H,6H-cyclopenta[c]pyrrolyl, wherein said ring system is optionally substituted by 1, 2, or 3 substituents independently selected from (1-6C)alkyl, (2-6C)alkenylene, (3-8C)cycloalkyl, oxo, C(O)OR₅, C(O)R₈ and phenyl, wherein said (1-6C)alkyl is optionally substituted by 1, 2, 3 or 4 substituents independently selected from halo, CF₃ and hydroxy, wherein each R₈ is independently selected from (1-6C)alkyl, (1-3C)alkoxy and CF₃, and wherein each R₅ is (1-6C)alkyl;
(10) R₁ and R₂, together with the nitrogen atom to which they are attached to, form a pyrrolyl ring, which is optionally substituted by 1, 2, or 3 substituents independently selected from (1-6C)alkyl, (2-6C)alkenylene, (3-8C)cycloalkyl, oxo, C(O)OR₅, C(O)R₈ and phenyl, wherein said (1-6C)alkyl is optionally substituted by 1, 2, 3 or 4 substituents independently selected from halo, CF₃ and hydroxy, wherein each R₈ is independently selected from (1-6C)alkyl, (1-3C)alkoxy and CF₃, and wherein each is (1-6C)alkyl;
(11) R₁ and R₂, together with the nitrogen atom to which they are attached to, form a pyrrolyl ring, which is optionally substituted by 1 or 2 substituents independently selected from (1-3C)alkyl, (2-4C)alkenylene, (3-6C)cycloalkyl, oxo, C(O)OR₅, C(O)R₈ and phenyl, wherein said (1-3C)alkyl is optionally substituted by 1 or 2 substituents independently selected from halo, CF₃ and hydroxy, wherein each R₈ is independently selected from (1-3C)alkyl, (1-3C)alkoxy and CF₃, arid wherein each is (1-6C)alkyl;
(12) R₁ and R₂, together with the nitrogen atom to which they are attached to, form a pyrrolyl ring, which is optionally substituted by 1 or 2 substituents independently selected from methyl, ethyl, cyclopropyl, phenyl, acetyl, methoxycarbonyl, propanoyl, trifluoroacetyl, 2,2,2-trifluoro-1-hydroxyethyl, 2-hydroxyisopropyl, prop-1-en-2-yl and phenyl;
(13) R₁ and R₂, together with the nitrogen atom to which they are attached to, form a pyrrolyl ring, which is optionally substituted by 1 or 2 substituents independently selected from methyl, cyclopropyl, acetyl, propanoyl, trifluoroacetyl, 2,2,2-trifluoro-1-hydroxyethyl, 2-hydroxyisopropyl and prop-1-en-2-yl;
(14) R₃ and R₄, together with the carbon atom to which they are attached to, form a 12, 13, 14, 15 or 16 membered tricyclic partially unsaturated heterocyclic or carbocyclic ring system, wherein said tricyclic ring system is optionally substituted by 1 or 2 substituents independently selected from (1-6C)alkyl, (2-6C)alkenylene, (2-6C)alkynylene, (3-8C)cycloalkyl, halo, oxo, hydroxy, cyano, amino, (1-3C)alkylamino, di₋[(1-3C)alkyl]-amino, CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃;
(15) R₃ and R₄, together with the carbon atom to which they are attached to, form a 12, 13, 14, 15 or 16 membered tricyclic partially unsaturated heterocyclic or carbocyclic ring system, wherein said tricyclic ring system is optionally substituted by 1 or 2 substituents independently selected from (1-6C)alkyl, halo and oxo;
(16) R₃ and R₄, together with the carbon atom to which they are attached to, form a 12, 13, 14, 15 or 16 membered tricyclic partially unsaturated carbocyclic ring system selected from the group consisting of 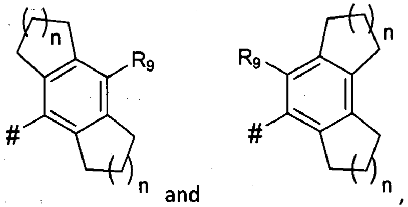 wherein # denotes the bond to the nitrogen atom of Formula (I); wherein n is an integer independently selected from 1, 2 and 3; and wherein R₉ is selected from the group consisting of hydrogen, (1-6C)alkyl, (2-6C)alkenylene, (2-6C)alkynylene, (3-8C)cycloalkyl, halo, oxo, hydroxy, cyano, amino, (1-3C)alkylamino, di-[(1-3C)alkyl]-amino, CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃;
(17) R₃ and R₄, together with the carbon atom to which they are attached to, form a 12, 13, 14, 15 or 16 membered tricyclic partially unsaturated carbocyclic ring system selected from the group consisting of 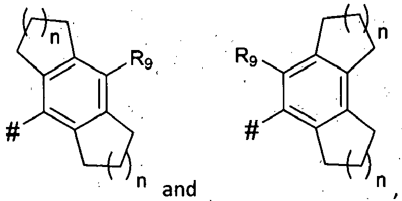 wherein # denotes the bond to the nitrogen atom of Formula (I); wherein n is an integer independently selected from 1, 2 and 3; and wherein R₉ is selected from the group consisting of hydrogen, (1-6C)alkyl, halo, CF₃ and OCF₃;
(18) R₃ and R₄, together with the carbon atom to which they are attached to, form a hexahydroindacene selected from the group consisting of 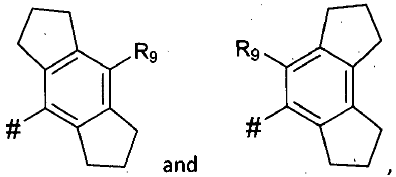 wherein # denotes the bond to the nitrogen atom of Formula (I); and wherein R₉ is selected from the group consisting of hydrogen, (1-6C)alkyl, (2-6C)alkenylene, (2-6C)alkynylene, (3-8C)cycloalkyl, halo, oxo, hydroxy, cyano, amino, (1-3C)alkylamino, di-[(1-3C)alkyl]-amino, CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃
(19) R₃ and R₄, together with the carbon atom to which they are attached to, form a hexahydroindacene selected from the group consisting of 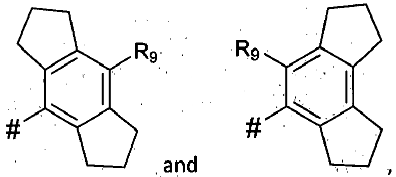 wherein # denotes the bond to the nitrogen atom of Formula (I); and wherein R₉ is selected from hydrogen, (1-6C)alkyl, halo, CF₃ and OCF₃;
(20) R₃ and R₄, together with the carbon atom to which they are attached to, form a hexahydroindacene ring: 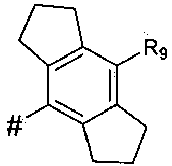 wherein # denotes the bond to the nitrogen atom of Formula (I); and wherein R₉ is selected from hydrogen, (1-6C)alkyl, halo, CF₃ and OCF₃; and
(21) R₃ and R₄, together with the carbon atom to which they are attached to, form an unsubstituted hexahydroindacene ring: 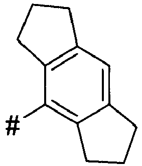 wherein # denotes the bond to the nitrogen atom of Formula (I).

A further particular group of compounds of the invention are compounds of Formula (I) above or pharmaceutically acceptable salt(s) thereof, wherein:-
R₁ and R₂ are suitably as defined in any one of paragraphs (1) to (13) above, particularly as defined in any one of paragraphs (5) to (13) above; and
R₃ and R₄ are suitably as defined in any one of paragraphs (14) to (21) above, particularly as defined in any one of paragraphs (16) to (21) above.

A particular group of compounds of the present invention are compounds of Formula (I), or pharmaceutically acceptable salt(s) thereof, wherein:-
R₁ and R₂, together with the nitrogen atom to which they are attached to, form a 5 membered monocyclic heteroaryl ring system selected from pyrrolyl, isoxazolyl, thiazolyl, isothiazolyl, pyrazolyl, imidazolyl, oxadiazolyl, thiadiazolyl, triazolyl and tetrazolyl; or R₁ and R₂, together with the nitrogen atom to which they are attached to, form a 8, 9 or 10 membered bicyclic heteroaryl ring system selected from indolyl, isoindolyl, indazolyl, 4,5,6,7-tetrahydroindolyl, 4,5,6,7-tetrahydroisoindolyl, benzimidazolyl, benzoxathiazolyl, benzoxadiazolyl, benzothiadiazolyl, quinolinyl, isoquinolinyl, purinyl, 1,8-naphthyridyl, pteridyl, 1H-pyrrolo[3,2-b]pyridinyl, 1H-pyrrolo[2,3-c]pyridinyl, 2H,4H,5H,6H-cyclopenta[c]pyrrolyl, pyrido[3,2-d]pyrimidyl arid pyridoimidazolyl,
wherein said monocyclic heteroaryl ring system or said bicyclic heteroaryl ring system is optionally substituted by 1, 2, or 3 substituents independently selected from (1-6C)alkyl, (2-6C)alkenylene, (2-6C)alkynylene, (3-8C)cycloalkyl, halo, nitro, cyano, CF₃, oxo, OR₅, N(R₆)(R₇), NR₅C(O)R₆, NR₆S(O)₂R₈, N(R₅)C(O)N(R₆)(R₇), S(O)₂R₈, S(O)R₈, S(O)(NR₅)(R₈), S(O)₂N(R₆)(R₇), C(O)OR₅, C(O)N(R₆)(R₇), C(O)R₈, C(NOR₅)(R₈), OC(O)N(R₆)(R₇), OC(O)R₈, phenyl, a 5 or 6 membered monocyclic heteroaryl ring system comprising 1, 2 or 3 heteroatoms independently selected from oxygen, nitrogen and sulfur, and a 3, 4, 5, or 6 membered monocyclic heterocyclyl ring system comprising 1 or 2 heteroatoms independently selected from oxygen, nitrogen and sulfur, wherein said (1-6C)alkyl, (2-6C)alkenylene, (2-6C)alkynylene, (3-8C)cycloalkyl, phenyl, 5 or 6 membered monocyclic heteroaryl ring system or 3, 4, 5, or 6 membered monocyclic heterocyclyl ring system is optionally substituted by 1, 2, 3 or 4 substituents independently selected from halo, cyano, oxo, CF₃, OR₅, N(R₆)(R₇), NR₅C(O)R₆, SR₅, C(O)N(R₆)(R₇), S(O)₂R₈, SOR₈, S(O)₂N(R₅)(R₆) and NR₅S(O)₂R₆;
R₃ and R₄, together with the carbon atom to which they are attached to, form a 12, 13, 14, 15 or 16 membered tricyclic partially unsaturated heterocyclic or carbocyclic ring system, wherein said tricyclic ring system is optionally substituted by 1 or 2 substituents independently selected from (1-6C)alkyl, (2-6C)alkenylene, (2-6C)alkynylene, (3-8C)cycloalkyl, halo, oxo, hydroxy, cyano, amino, (1-3C)alkylamino, di-[(1-3C)alkyl]-amino, CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃;
R₅, R₆ and R₇ are each independently selected from H, (1-6C)alkyl, CF₃, phenyl, a 5 or 6 membered monocyclic heteroaryl ring system comprising 1, 2 or 3 heteroatoms independently selected from oxygen, nitrogen and sulfur, a 3, 4, 5 or 6 membered monocyclic heterocyclyl ring system comprising 1 or 2 heteroatoms independently selected from oxygen, nitrogen and sulfur and a 3, 4, 5 or 6 membered saturated or partially unsaturated carbocyclic ring system, wherein said (1-6C)alkyl, phenyl, monocyclic heteroaryl ring, monocyclic heterocyclyl ring or carbocyclic ring system is optionally substituted with 1 or 2 substituents independently selected from halo, amino, methylamino, di(methyl)amino, nitro, hydroxy, methoxy, oxo, cyano, C(O)NH₂, C(O)NHCH₃, C(O)N(CH₃)(CH₃), CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃; and
each R₈ is independently selected from (1-6C)alkyl, CF₃, phenyl, a 5 or 6 membered monocyclic heteroaryl ring system comprising 1, 2 or 3 heteroatoms independently selected from oxygen, nitrogen and sulfur, a 3, 4, 5 or 6 membered monocyclic heterocyclyl ring system comprising 1 or 2 heteroatoms independently selected from oxygen, nitrogen and sulfur and a 3, 4, 5 or 6 membered saturated or partially unsaturated carbocyclic ring system, wherein said (1-6C)alkyl, phenyl, monocyclic heteroaryl ring, monocyclic heterocyclyl ring or carbocyclic ring system is optionally substituted with 1 or 2 substituents independently selected from halo, amino, methylamino, di(methyl)amino, nitro, hydroxy, methoxy, oxo, cyano, C(O)NH₂, C(O)NHCH₃, C(O)N(CH₃)(CH₃), CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃.

A further particular group of compounds of the present invention are compounds of Formula (I), or pharmaceutically acceptable salt(s) thereof, wherein:-
R₁ and R₂, together with the nitrogen atom to which they are attached to, form a ring system selected from pyrrolyl, indolyl, indazolyl, 4,5,6,7-tetrahydroisoindolyl, 1H-pyrrolo[3,2-b]pyridinyl, 1H-pyrrolo[2,3-c]pyridinyl and 2H,4H,5H,6H-cyclopenta[c]pyrrolyl, wherein said ring system is optionally substituted by 1, 2, or 3 substituents independently selected from (1-6C)alkyl, (2-6C)alkenylene, (2-6C)alkynylene, (3-8C)cycloalkyl, halo, cyano, CF₃, oxo, amino, (1-3C)alkylamino, di-[(1-3C)alkyl]-amino, OCF₃, S(O)₂CH₃, S(O)CH₃, S(O)₂NH₂, S(O)₂NHCH₃, S(O)₂N(CH₃)_{2,} NHS(O)₂CH₃ and N(CH₃)S(O)₂CH₃, OR₅, C(O)OR₅, C(O)R₈ and phenyl, wherein said (1-6C)alkyl, (2-6C)alkenylene, (2-6C)alkynylene, (3-8C)cycloalkyl or phenyl is optionally substituted by 1, 2, 3 or 4 substituents independently selected from halo, CF₃ and hydroxy, wherein each R₈ is independently selected from (1-6C)alkyl, (1-3C)alkoxy and CF₃, and wherein each R₅ is (1-6C)alkyl; and
R₃ and R₄, together with the carbon atom to which they are attached to, form a 12, 13, 14, 15 or 16 membered tricyclic partially unsaturated heterocyclic or carbocyclic ring system, wherein said tricyclic ring system is optionally substituted by 1 or 2 substituents independently selected from (1-6C)alkyl, halo and oxo.

A further particular group of compounds of the present invention are compounds of Formula (I), or pharmaceutically acceptable salt(s) thereof, wherein:-
R₁ and R₂, together with the nitrogen atom to which they are attached to, form a pyrrolyl ring, which is optionally substituted by 1 or 2 substituents independently selected from (1-3C)alkyl, (2-4C)alkenylene, (3-6C)cycloalkyl, oxo, C(O)OR₅, C(O)R₈ and phenyl, wherein said (1-3C)alkyl is optionally substituted by 1 or 2 substituents independently selected from halo, CF₃ and hydroxy, wherein each R₈ is independently selected from (1-3C)alkyl, (1-3C)alkoxy and CF₃, and wherein each R₅ is (1-6C)alkyl; and
R₃ and R₄, together with the carbon atom to which they are attached to, form a 12, 13, 14, 15 or 16 membered tricyclic partially unsaturated carbocyclic ring system selected from the group consisting of 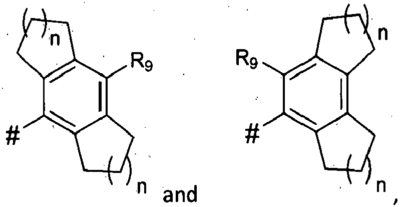 wherein # denotes the bond to the nitrogen atom of Formula (I); wherein n is an integer independently selected from 1, 2 and 3; and wherein R₉ is selected from the group consisting of hydrogen, (1-6C)alkyl, (2-6C)alkenylene, (2-6C)alkynylene, (3-8C)cycloalkyl, halo, oxo, hydroxy, cyano, amino, (1-3C)alkylamino, di-[(1-3C)alkyl]-amino, CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃.

A further particular group of compounds of the present invention are compounds of Formula (I), or pharmaceutically acceptable salt(s) thereof, wherein:-
R₁ and R₂, together with the nitrogen atom to which they are attached to, form a pyrrolyl ring, which is optionally substituted by 1 or 2 substituents independently selected from methyl, cyclopropyl, acetyl, propanoyl, trifluoroacetyl, 2,2,2-trifluoro-1-hydroxyethyl, 2-hydroxyisopropyl and prop-1-en-2-yl; and
R3 and R₄, together with the carbon atom to which they are attached to, form a hexahydroindacene ring: 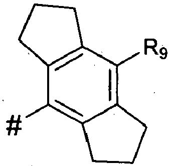 wherein # denotes the bond to the nitrogen atom of Formula (I); and wherein R₉ is selected from hydrogen, (1-6C)alkyl, halo, CF₃ and OCF₃.

A further particular group of compounds of the present invention are compounds of Formula (I), or pharmaceutically acceptable salt(s) thereof, wherein:-
R₁ and R₂, together with the nitrogen atom to which they are attached to, form a pyrrolyl ring, which is optionally substituted by 1 or 2 substituents independently selected from methyl, cyclopropyl, acetyl, propanoyl, trifluoroacetyl, 2,2,2-trifluoro-1-hydroxyethyl, 2-hydroxyisopropyl and prop-1-en-2-yl; and
R₃ and R₄, together with the carbon atom to which they are attached to, form an unsubstituted hexahydroindacene ring: 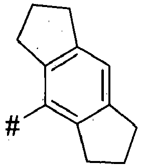 wherein # denotes the bond to the nitrogen atom of Formula (I).

A further particular group of compounds of the present invention are compounds of Formula (I), or pharmaceutically acceptable salt(s) thereof, wherein:-
R₁ and R₂, together with the nitrogen atom to which they are attached to, form a pyrrolyl ring, which is optionally substituted by 1 or 2 substituents independently selected from methyl, cyclopropyl, acetyl, trifluoroacetyl, 2,2,2-trifluoro-1-hydroxyethyl, 2-hydroxyisopropyl and prop-1-en-2-yl; and
R₃ and R₄, together with the carbon atom to which they are attached to, form an unsubstituted hexahydroindacene ring: 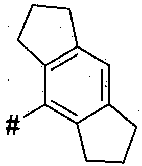 wherein # denotes the bond to the nitrogen atom of Formula (I).

A further particular group of compounds of the present invention are compounds of Formula (I), or pharmaceutically acceptable salt(s) thereof, wherein:-
R₁ and R₂, together with the nitrogen atom to which they are attached to, form a pyrrolyl ring, which is optionally substituted by acetyl, trifluoroacetyl and prop-1-en-2-yl; and R₃ and R₄, together with the carbon atom to which they are attached to, form an unsubstituted hexahydroindacene ring: 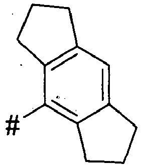 wherein # denotes the bond to the nitrogen atom of Formula (I).

Particular compounds of the invention are, for example, the sulfonylurea compounds of the Formula I that are disclosed within the Examples that are set out hereinafter.

For example, a particular compound of the invention is a compound of the Formula (I) selected form any one of the following:-
3-(1,2,3,5,6,7-hexahydro-s-indacen-4-yl)-1-(1H-pyrrole-1-sulfonyl)urea;
methyl 1-({[(1,2,3,5,6,7-hexahydro-s-indacen-4-yl)carbamoyl]amino}sulfonyl)-1H-pyrrole-3-carboxylate;
3-(1,2,3,5,6,7-hexahydro-s-indacen-4-yl)-1-{[3-(2-hydroxypropan-2-yl)-1H-pyrrol-1-yl]sulfony1}urea;
1-[(3-acetyl-1H-pyrrol-1-yl)sulfbnyl]-3-(1,2,3,5,6,7-hexahydro-s-indacen-4-yl)urea;
3-(1,2,3,5,6,7-hexahydro-s-indacen-4-yl)-1-{[3-(1-hydroxyethyl)-1H-pyrrol-1-yl]sulfonyl}urea;
3-(1,2,3,5,6,7-hexahydro-s-indacen-4-yl)-1-{[3-(prop-1-en-2-yl)-1H-pyrrol-1-yl]sulfonyl}urea;
3-(1,2;3,5,6,7-hexahydro-s-indacen-4-yl)-1-{[3-(trifluoroacetyl)-1H-pyrrol-1-yl]sulfonyl}urea;
3-(1,2,3,5,6,7=hexahydro-s-indaeen-4-yl)-1-(1H-indole-1-sulfonyl)urea;
3-(1,2,3,5,6,7-hexahydro-s-indacen-4-yl)-1-{1H-pyrrolo[3,2-b]pyridine-1-sulfonyl}urea;
3-(1,2,3,5,6,7-hexahydro-s-indacen-4-yl)-1-(1H-indazole-1-sulfonyl)urea;
1-[(3-cyclopropyl-1H-pyrrol-1-yl)sulfonyl]-3-(1,2,3,5,6,7-hexahydro-s-iridacen-4-yl)urea;
1-[(3-acetyl-1H-pyrrol-1-yl)sulfonyl]-3-(8-chloro-1,2,3,5,6,7-hexahydro-s-indacen-4-yl)urea;
3-(1,2,3,5,6,7-hexahydro-s-indacen-4-yl)-1-{1H-pyrrolo[2,3-c]pyridme-1-sulfbnyl}urea;
3-(1,2,3,5,6,7-hexahydro-s-indacen-4-yl)-1-[(3-nitro-1H-pyrrol-1-yl)sulfonyl]urea;
3-(1,2,3,5,6,7-hexahydro-s-indacen-4-yl)-1-[(3-propanoyl-1H-pyrrol-1-yl)sulfonyl]urea;
3-{1H,2H,3H,5H,6H,7H,8H-cyclopenta[b]naphthalen-4-yl}-1-(1H-indazole-1-sulfonyl)urea;
3-{1H,2H,3H,6H,7H,8H,9H-cyclopenta[a]naphthalen-5-yl}-1-(1H-indazole-1-sulfonyl)urea;
3-(1,2,3,5,6,7-hexahydro-s-indacen-4-yl)-1-({4-oxo-2H,4H,5H,6H-cyclopenta[c]pyrrol-2-yl}sulfonyl)urea;
1-[(3-benzoyl-1H-pyrrol-1-yl)sulfonyl]-3-(1,2,3,5,6,7-hexahydro-s-indacen-4-yl)urea;
3-(1,2,3,5,6,7-hexahydro-s-indacen-4-yl)-1-{[3-(2,2,2-trifluoro-1-hydroxyethyl)-1H-pyrrol-1-yl]sulfonyl}urea;
3-(1,2,3,6,7,8-hexahydroas-indacen-4-yl)-1-[3-(trifluoroacetyl)-1H-pyrrol-1-yl]sulfonyl}urea;
1-(6-oxo-1,2,3,6,7,8-hexahydroas-indacen-4-yl)-3-{[3-(trifluoroacetyl)-1H-pyrrol-1-yl]sulfonyl}urea;
3-(1,2,3,5,6,7-hexahydro-s-indacen-4-yl)-1-[(2-methyl-1H-pyrrol-1-yl)sulfonyl]urea;
1-[(3-acetyl-4-phenyl-1H-pyrrol-1-yl)sulfonyl]-3-(1,2,3,5,6,7-hexahydro-s-indacen-4-yl)urea; and
3-(1,2,3,5,6,7-hexahydro-s-indacen-4-yl)-1-[(4-oxo-4,5,6,7-tetrahydro-2H-isoindol-2-yl)sulfonyl]urea; or a pharmaceutically acceptable salt thereof.

According to a further aspect of the invention there is a sulfonylurea derivative of the Formula I, which is obtainable by following any of the Examples as disclosed herein.

A further feature is any of the scopes defined herein with the proviso that specific Examples, such as Example 1A, 1C, 2A, 2B etc. are individually disclaimed.

The various functional groups and substituents making up the compounds of the Formula (I) are typically chosen such that the molecular weight of the compound of the Formula (I) does not exceed 1000 daltons. More usually, the molecular weight of the compound will be less than 900, for example less than 800, or less than 750, or less than 700, or less than 650 daltons. More conveniently, the molecular weight is less than 600 and, for example, is 550 daltons or less.

A suitable pharmaceutically acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulfuric, phosphoric, trifluoroacetic, formic, citric methane sulfonate or maleic acid. In addition, a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a pharmaceutically acceptable cation, for example a salt with methylamine, dimethylamime, trimethylamine, piperidine, morpholine or tris-(2-hydroxyethyl)amine.

It will be understood that the compounds of Formula (I) and any pharmaceutically acceptable salts thereof, comprise stereoisomers, mixtures of stereoisomers, polymorphs of all isomeric forms of said compounds.

Compounds that have the same molecular formula but differ in the nature or sequence of bonding of their atoms or the arrangement of their atoms in space are termed "isomers". Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

The compounds of this invention may possess one or more asymmetric centers; such compounds can therefore be produced as individual (R)- or (S)-stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art (see discussion in Chapter 4 of "Advanced Organic Chemistry", 4th edition J. March, John Wiley and Sons, New York, 2001), for example by synthesis from optically active starting materials or by resolution of a racemic form. Some of the compounds of the invention may have geometric isomeric centres (E- and Z- isomers). It is to be understood that the present invention encompasses all optical, diastereoisomers and geometric isomers and mixtures thereof that possess inflammasome inhibitory activity.

The present invention also encompasses compounds of the invention as defined herein which comprise one or more isotopic substitutions. For example, H may be in any isotopic form, including 1H, 2H(D), and 3H (T); C may be in any isotopic form, including 12C, 13C, and 14C; and O may be in any isotopic form, including 16O and18O; and the like.

It is also to be understood that certain compounds of the Formula (I) may exist in solvated as well as unsolvated forms such as, for example, hydrated forms. A suitable pharmacutcially-acceptable solvate is, for example, a hydrate such as hemi-hydrate, a mono0hydrate, a di-hydrate or a tri-hydrate. It is to be understood that the invention encompasses all such solvated forms that possess inflammasome inhibitory activity.

It is also to be understood that certain compounds of the Formula (I) may exhibit polymorphism, and that the invention encompasses all such forms, or mixtures thereof, which possess inflammasome inhibitory activity. It is generally known that crystalline materials may be analysed using conventional techniques such as X-Ray Powder Diffraction analysis, Differential Scanning Calorimetry, Thermal Gravimetric Analysis, Diffuse Reflectance Infrared Fourier Transform (DRIFT) spectroscopy, Near Infrared (NIR) spectroscopy, solution and/or solid state nuclear magnetic resonance spectroscopy. The water content of such crystalline materials may be determined by Karl Fischer analysis.

Compounds of the Formula (I) may exist in a number of different tautomeric forms and references to compounds of the formula I include all such forms. For the avoidance of doubt, where a compound can exist in one of several tautomeric forms, and only one is specifically described or shown, all others are nevertheless embraced by Formula (I). Examples of tautomeric forms include keto-, enol-, and enolate-forms, as in, for example, the following tautomeric pairs: keto/enol (illustrated below), imine/enamine, amide/imino alcohol, amidine/amidine, nitroso/oxime, thioketone/enethiol, and nitro/aci-nitro. 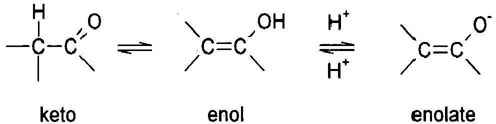 Compounds of the Formula (I) containing an amine function may also form N-oxides. A reference herein to a compound of the Formula I that contains an amine function also includes the N-oxide. Where a compound contains several amine functions, one or more than one nitrogen atom may be oxidised to form an N-oxide. Particular examples of N-oxides are the N-oxides of a *tert*iary amine or a nitrogen atom of a nitrogen-containing heterocycle. N-Oxides can be formed by treatment of the corresponding amine with an oxidizing agent such as hydrogen peroxide or a per-acid (e.g. a peroxycarboxylic acid), see for example Advanced Organic Chemistry, by Jerry March, 4th Edition, Wiley Interscience, pages. More particularly, N-oxides can be made by the procedure of L. W. Deady (Syn. Comm. 1977, 7, 509-514) in which the amine compound is reacted with m-chloroperoxybenzoic acid (mCPBA), for example, in an inert solvent such as dichloromethane.

The compounds of Formula (I) may be administered in the form of a pro-drug which is broken down in the human or animal body to release a compound of the invention. A pro-drug may be used to alter the physical properties and/or the pharmacokinetic properties of a compound of the invention. A pro-drug can be formed when the compound of the invention contains a suitable group or substituent to which a property-modifying group can be attached. Examples of pro-drugs include *in vivo* cleavable ester derivatives that may be formed at a carboxy group or a hydroxy group in a compound of the Formula (I) and in-vivo cleavable amide derivatives that may be formed at a carboxy group or an amino group in a compound of the Formula (I).

Accordingly, the present invention includes those compounds of the Formula (I) as defined hereinbefore when made available by organic synthesis and when made available within the human or animal body by way of cleavage of a pro-drug thereof. Accordingly, the present invention includes those compounds of the Formula I that are produced by organic synthetic means and also such compounds that are produced in the human or animal body by way of metabolism of a precursor compound, that is a compound of the Formula (I) may be a synthetically-produced compound or a metabolically-produced compound.

A suitable pharmaceutically acceptable pro-drug of a compound of the Formula (I) is one that is based on reasonable medical judgement as being suitable for administration to the human or animal body without undesirable pharmacological activities and without undue toxicity.

Various forms of pro-drug have been described, for example in the following documents :-
a) Methods in Enzymology, Vol. 42, p. 309-396, edited by K. Widder, et al. (Academic Press, 1985);
b) Design of Pro-drugs, edited by H. Bundgaard, (Elsevier, 1985);
c) A Textbook of Drug Design and Development, edited by Krogsgaard-Larsen and H. Bundgaard, Chapter 5 "Design and Application of Pro-drugs", by H. Bundgaard p. 113-191 (1991);
d) H. Bundgaard, Advanced Drug Delivery Reviews, 8, 1-38 (1992);
e) H. Bundgaard, et al., Journal of Pharmaceutical Sciences, 77, 285 (1988);
f) N. Kakeya, et al., Chem. Pharm. Bull., 32, 692 (1984);
g) T. Higuchi and V. Stella, "Pro-Drugs as Novel Delivery Systems", A.C.S. Symposium Series, Volume 14; and
h) E. Roche (editor), "Bioreversible Carriers in Drug Design", Pergamon Press, 1987.

A suitable pharmaceutically acceptable pro-drug of a compound of the Formula (I) that possesses a carboxy group is, for example, an *in vivo* cleavable ester thereof. An *in vivo* cleavable ester of a compound of the Formula (I) containing a carboxy group is, for example, a pharmaceutically acceptable ester which is cleaved in the human or animal body to produce the parent acid. Suitable pharmaceutically acceptable esters for carboxy include C1-6alkyl esters such as methyl, ethyl and *tert*-butyl, C1-6alkoxymethyl esters such as methoxymethyl esters, C1-6alkanoyloxymethyl esters such as pivaloyloxymethyl esters, 3-phthalidyl esters, C3-8cycloalkylcarbonyloxy- C1-6alkyl esters such as cyclopentylcarbonyloxymethyl and 1-cyclohexylcarbonyloxyethyl esters, 2-oxo-1,3-dioxolenylmethyl esters such as 5-methyl-2-oxo-1,3-dioxolen-4-ylmethyl esters and C1-6alkoxycarbonyloxy- C1-6alkyl esters such as methoxycarbonyloxymethyl and 1-methoxycarbonyloxyethyl esters.

A suitable pharmaceutically acceptable pro-drug of a compound of the Formula (I) that possesses a hydroxy group is, for example, an *in vivo* cleavable ester or ether thereof. An *in vivo* cleavable ester or ether of a compound of the Formula (I) containing a hydroxy group is, for example, a pharmaceutically acceptable ester or ether which is cleaved in the human or animal body to produce the parent hydroxy compound. Suitable pharmaceutically acceptable ester forming groups for a hydroxy group include inorganic esters such as phosphate esters (including phosphoramidic cyclic esters). Further suitable pharmaceutically acceptable ester forming groups for a hydroxy group include (1-10C)alkanoyl groups such as acetyl, benzoyl, phenylacetyl and substituted benzoyl and phenylacetyl groups, (1-10C)alkoxycarbonyl groups such as ethoxycarbonyl, N,N-(C1-6)2carbamoyl, 2-dialkylaminoacetyl and 2-carboxyacetyl groups. Examples of ring substituents on the phenylacetyl and benzoyl groups include aminomethyl, N-alkylaminomethyl, N,N-dialkylaminomethyl, morpholinomethyl, piperazin-1-ylmethyl and 4-(C1-4alkyl)piperazin-1-ylmethyl. Suitable pharmaceutically acceptable ether forming groups for a hydroxy group include α-acyloxyalkyl groups such as acetoxymethyl and pivaloyloxymethyl groups.

A suitable pharmaceutically acceptable pro-drug of a compound of the Formula (I) that possesses a carboxy group is, for example, an *in vivo* cleavable amide thereof, for example an amide formed with an amine such as ammonia, a C1-4alkylamine such as methylamine, a (C1-4alkyl)2amine such as dimethylamine, N-ethyl-N-methylamine or diethylamine, a C1-4alkoxy- C2-4alkylamine such as 2-methoxyethylamine, a phenyl-C1-4alkylamine such as benzylamine and amino acids such as glycine or an ester thereof.

A suitable pharmaceutically acceptable pro-drug of a compound of the Formula (I) that possesses an amino group is, for example, an *in vivo* cleavable amide derivative thereof. Suitable pharmaceutically acceptable amides from an amino group include, for example an amide formed with C1-10alkanoyl groups such as an acetyl, benzoyl, phenylacetyl and substituted benzoyl and phenylacetyl groups. Examples of ring substituents on the phenylacetyl and benzoyl groups include aminomethyl, N-alkylaminomethyl, N,N-dialkylaminomethyl, morpholinomethyl, piperazin-1-ylmethyl and 4-(C1-4alkyl)piperazin-1-ylmethyl.

The *in vivo* effects of a compound of the Formula (I) may be exerted in part by one or more metabolites that are formed within the human or animal body after administration of a compound of the Formula (I). As stated hereinbefore, the *in vivo* effects of a compound of the Formula (I) may also be exerted by way of metabolism of a precursor compound (a pro-drug).

Though the present invention may relate to any compound or particular group of compounds defined herein by way of optional, preferred or suitable features or otherwise in terms of particular embodiments, the present invention may also relate to any compound or particular group of compounds that specifically excludes said optional, preferred or suitable features or particular embodiments.

Suitably, the present invention excludes any individual compounds not possessing the biological activity defined herein.

### GENERAL METHODS OF PREPARATION

The compounds of the present invention can be prepared by any suitable technique known in the art. Particular processes for the preparation of these compounds are described further in the accompanying examples.

In the description of the synthetic methods described herein and in any referenced synthetic methods that are used to prepare the starting materials, it is to be understood that all proposed reaction conditions, including choice of solvent, reaction atmosphere, reaction temperature, duration of the experiment and workup procedures, can be selected by a person skilled in the art.

It is understood by one skilled in the art of organic synthesis that the functionality present on various portions of the molecule must be compatible with the reagents and reaction conditions utilised.

It will be appreciated that during the synthesis of the compounds of the invention in the processes defined herein, or during the synthesis of certain starting materials, it may be desirable to protect certain substituent groups to prevent their undesired reaction. The skilled chemist will appreciate when such protection is required, and how such protecting groups may be put in place, and later removed.

For examples of protecting groups see one of the many general texts on the subject, for example, 'Protective Groups in Organic Synthesis' by Theodora Green (publisher: John Wiley & Sons). Protecting groups may be removed by any convenient method described in the literature or known to the skilled chemist as appropriate for the removal of the protecting group in question, such methods being chosen so as to effect removal of the protecting group with the minimum disturbance of groups elsewhere in the molecule.

Thus, if reactants include, for example, groups such as amino, carboxy or hydroxy it may be desirable to protect the group in some of the reactions mentioned herein.

By way of example, a suitable protecting group for an amino or alkylamino group is, for example, an acyl group, for example an alkanoyl group such as acetyl, an alkoxycarbonyl group, for example a methoxycarbonyl, ethoxycarbonyl or t-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed by, for example, hydrolysis with a suitable base such as an alkali metal hydroxide, for example lithium or sodium hydroxide. Alternatively an acyl group such as a *tert*-butoxycarbonyl group may be removed, for example, by treatment with a suitable acid as hydrochloric, sulfuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium, sodium hydroxide or ammonia. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a t-butyl group which may be removed, for example, by treatment with an acid, for example an organic acid such as trifluoroacetic acid, or for example a benzyl group which may be removed, for example, by hydrogenation over a catalyst such as palladium-on-carbon.

The methodology employed to synthesise a compound of Formula (I) will vary depending on the nature of R₁, R₂, R₃, R₄ and any substituent groups associated therewith. Suitable processes for their preparation are described further in the accompanying Examples.

Once a compound of Formula (I) has been synthesised by any one of the processes defined herein, the processes may then further comprise the additional steps of:
(i) removing any protecting groups present;
(ii) converting the compound Formula (I) into another compound of Formula (I);
(iii) forming a pharmaceutically acceptable salt, hydrate or solvate thereof; and/or
(iv) forming a prodrug thereof.
An example of (ii) above is when a compound of Formula (I) is synthesised and then one or more of the groups of R₁, R₂, R₃, R₄, may be further reacted to change the nature of the group and provide an alternative compound of Formula (I).
The resultant compounds of Formula (I) can be isolated and purified using techniques well known in the art.

The starting materials for the preparation of compounds of the present invention can be prepared by methods as described in the examples or by methods known per se, as described in the literature of synthetic organic chemistry and known to the skilled person, or can be obtained commercially. The starting materials for the processes may, if desired, also be formed in situ by not isolating them from the reaction mixture, but instead immediately converting them further into the compounds of the invention or intermediate compounds. On the other hand, in general it is possible to carry out the reaction stepwise.

Conveniently, the reaction of the compounds is carried out in the presence of a suitable solvent, which is preferably inert under the respective reaction conditions. Examples of suitable solvents comprise but are not limited to hydrocarbons, such as hexane, petroleum ether, benzene, toluene or xylene; chlorinated hydrocarbons, such as trichlorethylene, 1,2-dichloroethane, tetrachloromethane, chloroform or dichloromethane; alcohols, such as methanol, ethanol, isopropanol, n-propanol, n-butanol or tert-butanol; ethers, such as diethyl ether, diisopropyl ether, tetrahydrofuran (THF) or dioxane; glycol ethers, such as ethylene glycol monomethyl or monoethyl ether or ethylene glycol dimethyl ether (diglyme); ketones, such as acetone or butanone; amides, such as acetamide, dimethylacetamide, dimethylformamide (DMF) or N-methylpyrrolidinone (NMP); nitriles, such as acetonitrile; sulfoxides, such as dimethyl sulfoxide (DMSO); nitro compounds, such as nitromethane or nitrobenzene; esters, such as ethyl acetate, or mixtures of the said solvents or mixtures with water.

The reaction temperature is suitably between about -100°C and 300°C, depending on the reaction step and the conditions used.

Reaction times are generally in the range between a fraction of a minute and several days, depending on the reactivity of the respective compounds and the respective reaction conditions. Suitable reaction times are readily determinable by methods known in the art, for example reaction monitoring. Based on the reaction temperatures given above, suitable reaction times generally lie in the range between 10 minutes and 48 hours.

Moreover, by utilizing the procedures described herein, in conjunction with ordinary skills in the art, additional compounds of the present invention can be readily prepared. Those skilled in the art will readily understand that known variations of the conditions and processes of the following preparative procedures can be used to prepare these compounds.

The present invention also refers to a process for manufacturing a compound according to Formula (I) or a pharmaceutically acceptable salt thereof, characterized in that:
a compound of Formula (II) 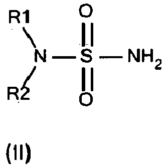 wherein, R₁ and R₂ are as defined herein for Formula (I);
is reacted with a compound of Formula (III) 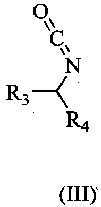 wherein, R₃ and R₄ are as defined herein for Formula (I);
to yield a compound of Formula (I) 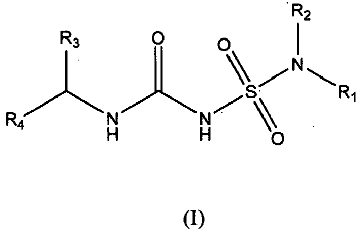 wherein, R₁, R₂, R₃ and R₄ are as defined herein for Formula (I).

As will be understood by the person skilled in the art of organic synthesis, compounds of the present invention are readily accessible by various synthetic routes, some of which are exemplified in the accompanying examples: The skilled person will easily recognize which kind of reagents and reactions conditions are to be used and how they are to be applied and adapted in any particular instance - wherever necessary or useful - in order to obtain the compounds of the present invention. Furthermore, some of the compounds of the present invention can readily be synthesized by reacting other compounds of the present invention under suitable conditions, for instance, by converting one particular functional group being present in a compound of the present invention, or a suitable precursor molecule thereof, into another one by applying standard synthetic methods, like reduction, oxidation, addition or substitution reactions; those methods are well known to the skilled person. Likewise, the skilled person will apply - whenever necessary or useful - synthetic protecting (or protective) groups; suitable protecting groups as well as methods for introducing and removing them are well-known to the person skilled in the art of chemical synthesis and are described, in more detail, in, e.g., P.G.M. Wuts, T.W. Greene, "Greene's Protective Groups in Organic Synthesis", 4th edition (2006) (John Wiley & Sons).

A particularly versatile starting point for making compounds of Formula (I) are precursor (or intermediate) molecules of Formula (II). Compounds of Formula (II) are readily available as shown in Scheme 1 below.

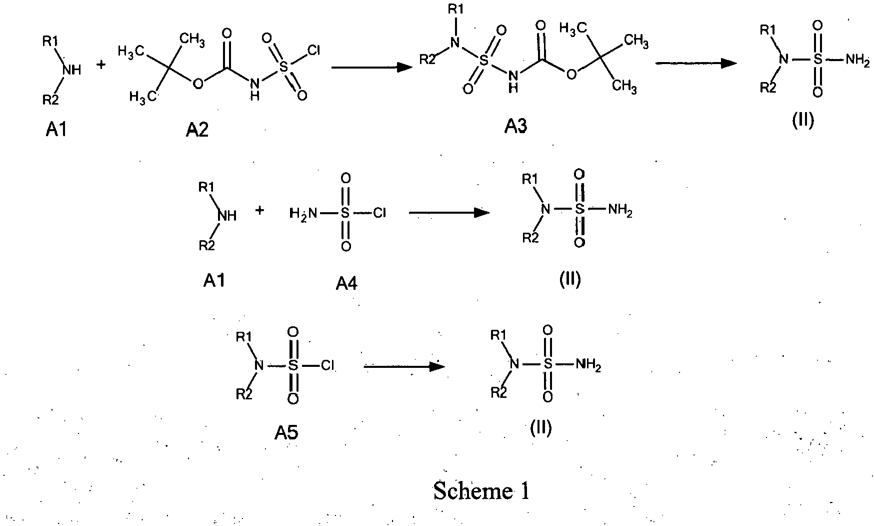

One starting point could be the N-H containing heteroaryl ring (A1) which is reacted with tert-butyl N-(chlorosulfonyl)carbamate (A2) under suitable reaction conditions to form corresponding protected sulfonamide (A3) which after deprotection step forms sulfonamide of Formula (II). N-H containing heteroaryls (A1) are either commercially available or can be prepared by utilizing known synthetic methodologies. Alternatively, sulfonamides (II) can be prepared using reaction of the N-H containing heteroaryl (A1) with unprotected sulfamoyl chloride (A4) under suitable reaction conditions. Additionally, sulfonamides (II) can be prepared using reaction of the of N,N-disubstituted sulfamoyl chloride (A5) with ammonia under suitable reaction conditions. N,N-disubsfituted sulfamoyl chlorides R₁R₂NSO₂Cl (A5) are either commercially available or can be prepared by utilizing known synthetic methodologies.

Compounds of formula (III) can be obtained by reacting a suitably secondary amine (A6) with phosgene or triphosgene under suitable reaction conditions to form isocyanates (III), as shown in Scheme 2 below.

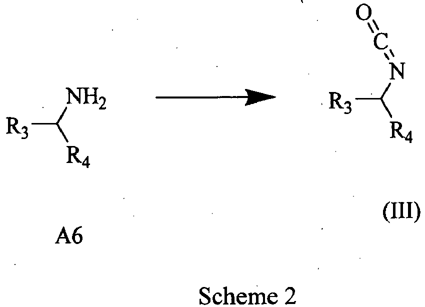

In a subsequent reaction step the isocyanate compound of Formula (III) and the sulfonamide of Formula (II) are converted to the corresponding sulfonylurea compound of Formula (I) by reacting (II) and (III) in the presence of base under suitable reaction conditions, as shown in Scheme 3 below.

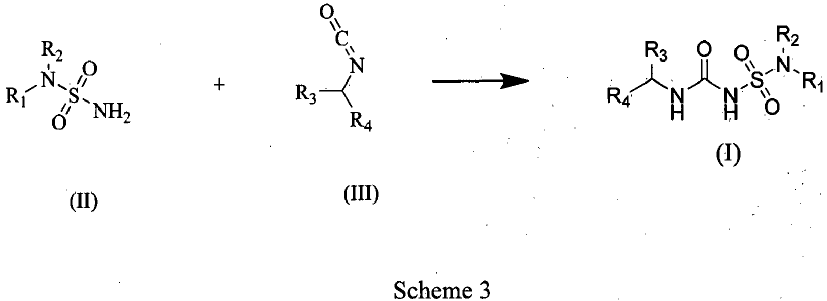

Compounds of the present invention can be further modified in order to afford further compounds of formula (I). For instance, compounds of formula (I) bearing a ketone group at one of the substituents R¹, R², R³ and/or R⁴ may be converted into the respective alcohols or prop-1-en-2-yl derivatives by reacting the ketone with suitable reagents, e.g. NaBH₄ or methyl magnesium chloride respectively.

Certain of the intermediates defined herein may be novel and these are provided as a further feature of the invention.

### PHARMACEUTICAL COMPOSITIONS

According to a further aspect of the invention there is provided a pharmaceutical composition which comprises a compound of the invention as defined hereinbefore, or a pharmaceutically acceptable salt, hydrate or solvate thereof, in association with a pharmaceutically acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular, intraperitoneal or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

An effective amount of a compound of the present invention for use in therapy is an amount sufficient to treat or prevent an inflammasome related condition referred to herein, slow its progression and/or reduce the symptoms associated with the condition.

The amount of active ingredient that is combined with one or more excipients to produce a single dosage form will necessarily vary depending upon the individual treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the Formula I will naturally vary according to the nature and severity of the conditions, the age and sex of the animal or patient and the route of administration, according to well-known principles of medicine.

In using a compound of the invention for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous or intraperitoneal administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration may also be suitable, particularly in tablet form. Typically, unit dosage forms will contain about 0.5 mg to 0.5 g of a compound of this invention.

### THERAPEUTIC USES AND APPLICATIONS

The present invention provides compounds that function as inhibitors of inflammasome activity. The present invention therefore provides a method of inhibiting inflammasome activity *in vitro* or *in vivo,* said method comprising contacting a cell with an effective amount of a compound, or a pharmaceutically acceptable salt thereof, as defined herein.

The present invention also provides a method of treating a disease or disorder in which inflammasome activity is implicated in a patient in need of such treatment, said method comprising administering to said patient a therapeutically effective amount of a compound, or a pharmaceutically acceptable salt thereof, or a pharmaceutical composition as defined herein.

On a general level, the compounds of the present invention, which inhibit the maturation of cytokines of the IL-1 family, are effective in all therapeutic indications that are mediated or associated with elevated levels of active forms of cytokines belonging to IL-1 family of cytokines (Sims J. et al. Nature Reviews Immunology 10, 89-102 (February 2010).

Exemplary diseases and the corresponding references will be given in the following: autoinflammatory and autoimmune diseases like CAPS (Dinarello CA. Immunity. 2004 Mar;20(3):243-4; Hoffman HM. al. Reumatología 2005; 21(3)), gout, rheumatoid arthritis (Gabay C et al. Arthritis Research & Therapy 2009, 11:230; Schett G. et al. Nat Rev Rheumatol. 2016 Jan;12(1):14-24.), Crohn's disease (Jung Mogg Kim Korean J Gastroenterol Vol. 58 No. 6, 300-310), COPD (Mortaz E. et al. Tanaffos. 2011; 10(2): 9-14.), fibrosis (Gasse P. et al. Am J Respir Crit Care Med. 2009 May 15;179(10):903-13), obesity, type 2 diabetes ((Dinarello CA. et al. Curr Opin Endocrinol Diabetes Obes. 2010 Aug;17(4):314-21)) multiple sclerosis (see EAE-model in Coll RC. et al. Nat Med. 2015 Mar;21(3):248-55) and many others (Martinon F. et al. Immunol. 2009. 27:229-65) like Parkinson disease or Alzheimer disease (Michael T. et al. Nature 493, 674-678 (31 January 2013); Halle A. et al., Nat Immunol. 2008 Aug;9(8):857-65; Saresella M. et al. Mol Neurodegener. 2016 Mar 3;11:23) and some oncological disorders.

As mentioned above, inflammasome activity is implicated in a wide range of disorders and a compound of Formula I may therefore be useful as a therapeutic agent for a number of indications including, but not limited to, cryopyrin-associated autoinflammatory syndromes (CAPS) including familial cold autoinflammatory syndrome (FCAS), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), gout, rheumatoid arthritis, Crohn's disease, COPD, fibrosis, obesity, type 2 diabetes, multiple sclerosis and neuro-inflammation occurring in protein miss-folding diseases (such as Prion diseases), Parkinson's disease, Alzheimer's disease, cancers (such as melanoma, sarcoma, colorectal cancer, myeloma mesothelioma, lung cancer and breast cancer), meningitis, salpingitis, septic shock, disseminated intravascular coagulation, adult respiratory distress syndrome, acute or chronic inflammation, cholangitis, colitis, encephalitis, endocarditis, glomerulonephritis, hepatitis, myocarditis, pancreatitis, pericarditis, reperfusion injury, vasculitis, acute and delayed hypersensitivity, graft rejection, and graft-versus-host disease, periodonate diseases, interstitial pulmonary fibrosis, cirrhosis, systemic sclerosis, keloid formation, cachexia, percussion injury, depression, atherosclerosis (including cardiomyopathy, myocarditis and heart failure), osteoporosis and Adult and Juvenile Onset Still's Disease.

Suitably, the compounds according to the present invention can be used for the treatment of a disease selected from the group consisting of an autoinflammatory disease, an autoimmune disease, a neurodegenerative disease and cancer. Said autoinflammatory and autoimmune disease is suitably selected from the group consisting of a cryopyrin-associated autoinflammatory syndrome (CAPS) such as for example familial cold autoinflammatory syndrome (FCAS), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), gout, rheumatoid arthritis, Crohn's disease, COPD, fibrosis, obesity, type 2 diabetes, multiple sclerosis and neuro-inflammation occurring in protein miss-folding diseases, such as Prion diseases. Said neurodegenerative disease is suitably selected from Parkinson's disease and Alzheimer's disease.

Accordingly, the compounds of the present invention can be used for the treatment of a disease selected from the group consisting of cryopyrin-associated autoinflammatory syndrome (CAPS) such as for example familial cold autoinflammatory syndrome (FCAS), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), gout, rheumatoid arthritis, Crohn's disease, COPD, fibrosis, obesity, type 2 diabetes, multiple sclerosis, neuro-inflammation occurring in protein miss-folding diseases, such as Prion diseases, Parkinson's disease, Alzheimer's disease and cancer.

Compounds of the present invention, or pharmaceutically acceptable salts thereof, may be administered alone as a sole therapy or can be administered in addition with one or more other substances and/or treatments. Such conjoint treatment may be achieved by way of the simultaneous, sequential or separate administration of the individual components of the treatment.

For example, therapeutic effectiveness may be enhanced by administration of an adjuvant (i.e., by itself the adjuvant may only have minimal therapeutic benefit, but in combination with another therapeutic agent, the overall therapeutic benefit to the individual is enhanced). Alternatively, by way of example only, the benefit experienced by an individual may be increased by administering the compound of Formula (I) with another therapeutic agent (which also includes a therapeutic regimen) that also has therapeutic benefit.

In the instances where the compound of the present invention is administered in combination with other therapeutic agents, the compound of the invention may need not be administered via the same route as other therapeutic agents, and may, because of different physical and chemical characteristics, be administered by a different route. For example, the compound of the invention may be administered orally to generate and maintain good blood levels thereof, while the other therapeutic agent may be administered intravenously. The initial administration may be made according to established protocols known in the art, and then, based upon the observed effects, the dosage, modes of administration and times of administration can be modified by the skilled clinician.

The particular choice of other therapeutic agent will depend upon the diagnosis of the attending physicians and their judgment of the condition of the individual and the appropriate treatment protocol. According to this aspect of the invention there is provided a combination for use in the treatment of a disease in which inflammasome activity is implicated comprising a compound of the invention as defined hereinbefore, or a pharmaceutically acceptable salt thereof, and another suitable agent.

According to a further aspect of the invention there is provided a pharmaceutical composition which comprises a compound of the invention, or a pharmaceutically acceptable salt thereof, in combination with a suitable, in association with a pharmaceutically acceptable diluent or carrier.

In addition to its use in therapeutic medicine, compounds of Formula (I) and pharmaceutically acceptable salts thereof are also useful as pharmacological tools in the development and standardization of *in vitro* and *in vivo* test systems for the evaluation of the effects of inhibitors of inflammasome in laboratory animals such as cats, dogs, rabbits, monkeys, rats and mice, as part of the search for new therapeutic agents.

In any of the above-mentioned pharmaceutical composition, process, method, use, medicament, and manufacturing features of the instant invention, any of the alternate embodiments of macromolecules of the present invention described herein also apply.

### ROUTES OF ADMINISTRATION

The compounds of the invention or pharmaceutical compositions comprising these compounds may be administered to a subject by any convenient route of administration, whether systemically/ peripherally or topically (i.e., at the site of desired action).

Routes of administration include, but are not limited to, oral (e.g. by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eye drops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intra-arterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly.

### EXAMPLES

The invention will now be described with reference to the following illustrative examples. Some abbreviations that may appear in this section are defined as follows:
*t*-BuOH - 2-Methylpropan-2-ol
ACN - Acetonitrile
DCM - Dichloromethane
DIPEA - Ethyldiisopropylamine
DMF - N,N-Dimethylformamide
DMA - Dimethylacetamide
DMSO - Dimethyl sulfoxide
EtOAc - Ethyl acetate
Et₂O - Diethyl ether
HCl - Hydrochloric acid solution
HPLC - High-performance liquid chromatography
FCC - Flash column chromatography
KHSO₄ - Potassium bisulfate
KOtBu - Potassium tert-butoxide
LC - Liquid chromatography
MeOH - Methanol
MS - Mass spectrometry
Na₂SO₄ - Sodium sulfate
NH₄Cl - Ammonium chloride
NaBH₄ - Sodium borohydride
NaH - Sodium hydride
NaOH - Sodium hydroxide
NMR - Nuclear magnetic resonance
RM - reaction mixture
rt - room temperature
TEA - Triethylamine
TFA - Trifluoroacetic acid
THF - Tetrahydrofuran
TLC - Thin-layer chromatography
TTIP - Titanium (IV) isopropoxide
UPLC - Ultra performance liquid chromatography

The compounds of the present invention can be prepared according to the procedures of the following Schemes and Examples, using appropriate materials and are further exemplified by the following specific examples. Analytical data of compounds made according to the following examples are shown in the accompanying tables.

Unless otherwise specified, all starting materials are obtained from commercial suppliers and used without further purifications. Unless otherwise specified, all temperatures are expressed in °C and all reactions are conducted at rt. Compounds are purified by either silica chromatography or preparative HPLC.

¹H NMR is recorded on 400 MHz spectrometers. Chemical shifts (δ) are reported in ppm relative to the residual solvent signal (δ= 2.5 ppm for 1H NMR in DMSO-d6). ¹H NMR data are reported as follows: chemical shift (multiplicity, coupling constants and number of hydrogens). Multiplicity is abbreviated as follows: s (singlet), d (doublet), t (triplet), q (quartet), m (multiplet), br (broad).

### LC-MS analyses

### UPLC-MS:

Equipment: Shimadzu LC-MS 2020 column: Waters Acquity UPLC HSS C18, 50 mm x 2.1 mm x 1.8 µm
Eluents:
(A) 0.1% formic acid in ACN
(B) 0.1% formic acid in water
Autosampler: injection volume: 1µL
Pump:

| **Time [min]** | **Flow [*mL*/min]** | **% B** |
|---|---|---|
| 0.00 | 0.5 | 95 |
| 0.00 | 0.5 | 95 |
| 4.00 | 0.5 | 5 |
| 5.00 | 0.5 | 5 |
| 5.20 | 0.5 | 95 |
| 6.00 | 0.5 | 95 |

Column compartment:
- column temperature: 25 °C
- time of analysis: 6 min
Detector:
- wave lengths: 254, 230, 270, 280 nm

### HPLC-MS:

Equipment: MS Bruker Amazon SL; LC Dionex Ultimate 3000; HPLC with UV-Vis or DAD detector
column: Kinetex XB C18 4.6x50mm 2.6µm Eluents:
(A) 0.1% formic acid-water solution
(B) 0.1% formic acid- ACN solution
Autosampler: injection volume: 1µL
Pump: flow: 0.5mL/min

| **Time [min]** | **[%] B** |
|---|---|
| 0.0 | 20 |
| 6.7 | 80 |
| 7.5 | 80 |
| 7.8 | 95 |
| 9.5 | 95 |
| 10.0 | 20 |
| 12.0 | 20 |

Column compartment:
- column temperature: 25°C
- time of analysis: 12min
Detector:
wavelength: 220, 254, 280 nm

### Synthetic Examples

### Example 1A

### 3-(1,2,3,5,6,7-Hexahydro-s-indacen-4-yl)-1-(1H-indole-1-sulfonyl)-1-sodiourea

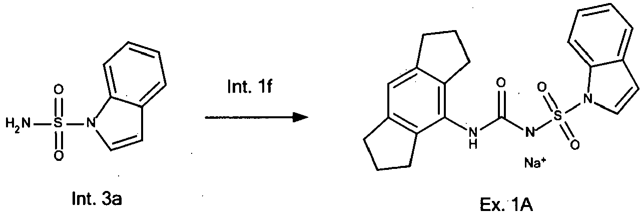

NaH (60% in mineral oil) (0.024 g, 0.61 mmol) was added to a solution of 1H-indole-1-sulfonamide (Intermediate 3a) (0.1 g, 0.51 mmol) in anhydrous DMF (2 mL). After stirring for 10 min, solution of 4-isocyanato-1,2,3,5,6,7-hexahydro-s-indacene (Intermediate 1f) (0.107 g, 0.54 mmol) in anhydrous DMF (1 mL) was added. The RM was stirred at rt for overnight. Then solvent was evaporated at reduced pressure. The residue was treated with 2-propanol, solids were filtered off and washed with Et₂O, hexane. The product was obtained as a white solid (0.035 g, 0.084 mmol, 16.4 %). HPLC-MS: purity 97.4%, 396.4 [M+H]⁺. 1H NMR (400 MHz, DMSO-d6) δ 7.93 (d, J = 8.2 Hz, 1H), 7.67 (s, 1H), 7.58 - 7.45 (m, 2H), 7.11 (t, J = 7:6 Hz, 1H), 7.03 (t, J = 7.4 Hz, 1H), 6.76 (s, 1H), 6.34 (d, J = 3.4 Hz, 1H), 2.72 (t, J = 7.4 Hz, 4H), 2.56 (t, J = 7.4 Hz, 4H), 1.85 (p, J = 7.4 Hz, 4H).

The following compounds were prepared using analogous procedures to those described in Example 1A, using the appropriate starting materials:

| **Ex. No.** | **Name and structure** | **NMR** | **LC-MS** | **Starting materials (SM) and yield** |
|---|---|---|---|---|
| 1B | 1-[(3-acetyl-1H-pyrrol-1-yl)sulfonyl]-3-(1,2,3,5,6,7-hexahydro-s-indacen-4-yl)-1-sodiourea | 1H NMR (400 MHz, DMSO-d6) 8 7.85 (s, 1H), 7.80 (t, J = 1.8 Hz, 1H), 7.09 (dd, J = 3.0,2.0 Hz, 1H), (s, 1H), 6.36 (dd, J = 3.0, 1.8 Hz, 1H), 2.80 - 2.71 (m, 4H), 2.70 - 2.60 (m, 4H), 2.31 (s, 3H), 1.96 - 1.84 (m, 4H). | [M-H]⁻ =386.1 | Intermediates 3b and 1f. Yield: 0.12 g, 0.29 mmol, 63%; white solid. |
| | 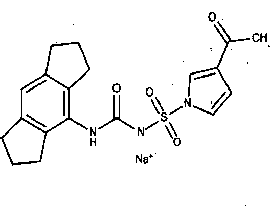 | | | |
| 1C | methyl 1-({[(1,2,3,5,6,7-hexahydro-s-indacen-4-yl)carbamoyl](sodio)ami no}sulfonyl)-1H-pyrrole-3-carboxylate | 1H NMR (400 MHz, DMSO-d6) δ 7.84 (s, 1H), 7.69 (s, 1H), 7.07 (s, 1H), 6.81 (s, 1H), 6.35 (s, 1H), 3.70 (s, 3H), 2.76 (t, J = 7.5 Hz, 4H), 2.65 (t, J = 7.5 Hz, 4H), 1.90 (t, J = 7.4 Hz, 4H). | [M-H]⁻ =402.1 | Intermediates 3c and 1f. Yield: 0.12 g, 0.28 mmol, 50%; white solid. |
| | 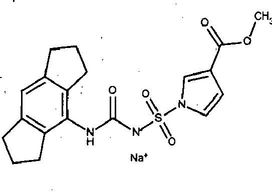 | | | |
| 1D | 3-(1,2,3,5,6,7-hexahydro-s-indacen-4-yl)-1-{1H-pyrrolo[3,2-b]pyridine-1-sulfonyl}-1-sodiourea | 1H NMR (400 MHz, DMSO-d6) δ 8.32 (s, 1H), 8.19 (s, 1H), 7.80 (s, 1H), 7.75 (s, 1H), 7.13 (s, 1H), 6.76 (s, 1H), 6.48 (s, 1H), 2.71 (br s, 4H), 2.53 (br s, 4H), 1.84 (br s, 4H). | [M+H]⁺ =397.6 | Intermediates 3d and 1f. Yield: 0.04 g, 0.10 mmol, 45%; white solid. |
| | 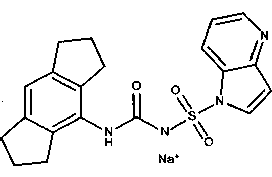 | | | |
| 1E | 3-(1,2,3,5,6,7-hexahydro-s-indacen-4-yl)-1-(1H-indazole-1-sulfonyl)-1-sodiourea | 1H NMR (400 MHz, DMSO-d6) δ 8.05 (d, J = 11.8 Hz, 2H), 7.86 (s, 1H), 7.73 (d, J = 8.0 Hz, 1H), 7.37 (t, J = 7.7 Hz, 1H), 7.16 (t, J = 7.5 Hz, 1H), 6.77 (s, 1H), 2.74 - 2.69 (m, 4H), 2.55 (t, J = 7.3 Hz, 4H), 1.85 (p, J = 7.4 Hz, 4H). | [M-H]⁻ =395.2 | Intermediates 4a and 1f. Yield: 0.007 g, 0.016 mmol, 6.3%, beige solid. |
| | 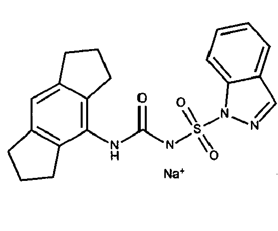 | | | |
| 1F | 1-[(3-cyclopropyl-1H-pyrrol-1-yl)sulfonyl]-3-(1,2,3,5,6,7-hexahydro-s-indacen-4-yl)-1-sodiourea | 1H NMR (400 MHz, DMSO-d6) δ 7.66 (s, 1H), 6.91 (t, J = 2.6 Hz, 1H), 6.84 (t, J = 2.0 Hz, 1H), 6.79 (s, 1H), 5.81 - 5.66 (m, 1H), 2.78 - 2.73 (m, 4H), 2.65 (t, J = 7.3 Hz, 4H), 1.91 (p, J = 7.5 Hz, 4H), 1.67 - 1.58 (m, 1H), 0.78 - 0.66 (m, 2H), 0.43 - 0.34 (m, 2H). | [M-H]⁻ =384.1 | Intermediates 5a and 1f. Yield: 0.006 g; 0.415 mmol; 8.5 %, yellow solid. |
| | 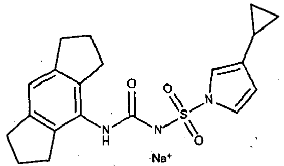 | | | |
| 1G | 3-(1,2,3,5,6,7-hexahydro-s-indacen-4-yl)-1-[(3-propanoyl-1H-pyrrol-1 - yl)sulfonyl]-1-sodiourea | 1H NMR (400 MHz, DMSO-d6) δ 7.85 (s, 1H), 7.79 (t, J = 1.9 Hz, 1H), 7.09 (dd, J = 3.1,2.0 Hz, 1H), 6.81 (s, 1H), 6.37 (dd, J = 3.1, 1.8 Hz, 1H), 2.82 - 2.57 (m, 10H), 1.90 (p, J = 7.3 Hz, 4H), 1.04 (t, J = 7.4 Hz, 3H). | [M-H]⁻ =400.3 | Intermediates 5b and If. Yield: 0.075 g; 0.177 mmol; 57 %, white solid. |
| | 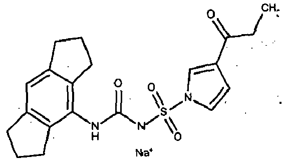 | | | |
| 1H | 3-(1,2,3,5,6,7-hexahydro-s-indacen-4-yl)-1-{1H-pyrrolo[2,3-c]pyridine-1-sulfonyl}-1-sodiourea | 1H NMR (400 MHz, DMSO-d6) δ 9.15 (s, 1H), 8.12 (d, J = 5.3 Hz, 1H), 7.78 (s, 1H), 7.70 (d, J = 3.3 Hz, 1H), 7.48 (dd, J = 5.4, 1.1 Hz, 1H), 6.76 (s, 1H), 6.42 (d, J = 3.3 Hz, 1H), 2.71, (t, J = 7.4 Hz, 4H), 2.54 (s, 4H), 1.84 (p, J = 7.4 Hz, 4H). | [M+H]⁺ =397.7 | Intermediates 3e and 1f. Yield: 0.015 g; 0.037 mmol; 60 %, white solid. |
| | 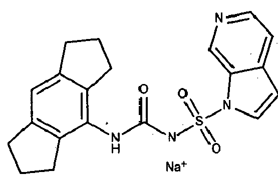 | | | |
| 11 | 1-[(3-acetyl-1H-pyrrol-1-yl)sulfonyl]-3-(8-chloro-1,2,3,5,6,7-hexahydro-s-indacen-4-yl)-1-sodiourea | 1H NMR (400 MHz, DMSO-d6) δ 7.93 (s, 1H), 7.79 (t, J = 1.9 Hz, 1H), 7.08 (dd, J = 3.1, 2.0 Hz, 1H), 6.36 (dd, J= 3.1, 1.8 Hz, 1H), 2.81 (t, J = 7.5 Hz, 4H), 2.75 (t, J = 7.4 Hz, 4H), 2.30 (s, 3H), 1.96 (p, J = 7.3 Hz, 4H). | [M-H]⁻ =420.1 | Intermediates 6b and 3b. Yield: 0.025 g; 0.059 mmol; 43 %, white solid. |
| | 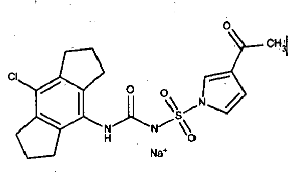 | | | |
| 1J | 3-(1,2,3,5,6,7-hexahydro-s-indacen-4-yl)-1-[(3-nitro-1H-pyrrol-1-yl)sulfonyl]-1-sodiourea | 1H NMR (400 MHz, DMSO-d6) δ 8.00 (br. s, 2H), 7.12 (dd, J = 3.4, 2.3 Hz, 1H), 6.82 (s, 1H), 6.63 (dd, J = 3.4, 1.9 Hz, 1H), 2.76 (t, J = 7.4 Hz, 4H), 2.64 (t, J = 7.4 Hz, 4H), 1.91 (p, J = 7.4 Hz, 4H). | [M-H]⁻ =389.1 | Intermediates 3f and 1f. Yield: 0.068g; 0.166 mmol; 68 %, light yellow solid. |
| | 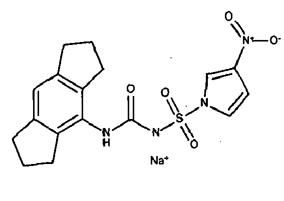 | | | |
| 1K | 3-(1,2,3,5,6,7-hexahydro-s-indacen-4-yl)-1-(1H-pyrrole-1-sulfonyl)-1-sodiourea | 1H NMR (400 MHz, DMSO-d6) δ 7.67 (s, 1H), 7.07 (t, J = 2.2 Hz, 2H), 6.79 (s, 1H), 5.96 (t, J = 2.2 Hz, 2H), 3.52 (s, 1H), 2.76 (t, J = 7.4 Hz, 5H), 2.65 (t, J = 7.4 Hz, 4H), 1.90 (p, J = 7.4 Hz, 4H). | [M-H]⁻ =344.1 | Intermediates 3g and 1f. Yield: 0.033 g; 0.09 mmol; 22 %, light yellow solid. |
| | 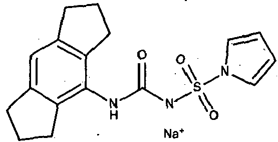 | | | |
| 1L | 3-(1,2,3,5,6,7-hexahydro-s-indacen-4-yl)-1-sodio-1-{[3-(trifluoroacetyl)-1H-pyrrol-1-yl]sulfonyl}urea | 1H NMR (400 MHz, DMSO-d6) δ 8.01 (q, J = 1.7 Hz, 1H), 7.98 (s, 1H), 7.25 (dd, J = 3.2, 1.9 Hz, 1H), 6.82 (s, 1H), 6.60 (dd, J = 3.2, 1.8 Hz, 1H), 2.76 (t, J = 7.4 Hz, 4H), 2.64 (t, J = 7.4 Hz, 4H), 1.90 (p, J = 7.4 Hz, 4H). | [M-H]⁻ =440.0 | Intermediates 3h and If. Yield: 0.04 g; 0.086 mmol; 40 %, white solid. |
| | 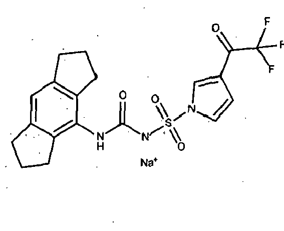 | | | |
| 1M | 3-{1H,2H,3H,5H,6H,7H,8H -cyclopenta[b]naphthalen-4-yl}-1-(1H-indazole-1-sulfonyl)-1-sodiourea | 1H NMR (400 MHz, DMSO-d6) δ 8.13 - 7.90 (m, 2H), 7.72 (d, J = 8.7 Hz, 1H), 7.59 (s, 1H), 7.36 (t, J = 7.3 Hz, 1H), 7.15 (t, J = 7.4 Hz, 1H), 6.67 (s, 1H), 2.79 - 2.66 (m, 2H), 2.65 - 2.56 (m, 2H), 2.48 - 2.30 (m, 4H), 1.89 - 1.70 (m, 2H), 1.66 - 1.43 (m; 4H). | [M-H]⁻ =409.1 | Intermediates 4a and 1j. Yield: 0.02 g; 0.048 mmol; 50 %, beige solid. |
| | 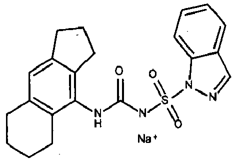 | | | |
| 1N | 3-{1H,2H,3H,6H,7H,8H,9H -cyclopenta[a]naphthalen-5-yl}-1-(H-indazole-1-sulfonyl)-1-sodiourea | 1H NMR (400 MHz, DMSO-d6) δ 8.13 - 8.00 (m, 2H), 7.72 (d, J = 7.9 Hz, 1H), 7.48 (s, 1H), 7.38 (t, J = 7.0 Hz, 1H), 7.26 - 7.11 (m, 2H), 3.45 - 3.33 (m, 2H), 2.70 (t, J = 6.7 Hz, 2H), 2.62 (t, J = 7.1 Hz, 2H), 2.44 (s, 2H), 1.99 - 1.86 (m, 2H), 1.72 - 1.55 (m, 4H). | [M-H]⁻ =409.1 | Intermediates 4a and 1k. Yield: 0.01 g; 0.024 mmol; 35 %, beige solid. |
| | 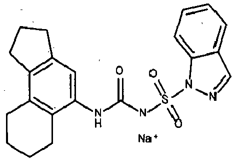 | | | |
| 10 | 3-(1,2,3,5,6,7-hexahydro-s-indacen-4-yl)-1-({4-oxo-2H,4H,5H,6H-cyclopenta[c]pyrrol-2-yl}sulfonyl)-1-sodiourea | 1H NMR (400 MHz, DMSO-d6) δ 7.83 (s, 1H), 7.38 (d, J = 1.5 Hz, 1H), 6.94 (s, 1H), 6.81 (s, 1H), 2.83 - 2.60 (m, 12H), 1.91 (p, J = 7.4 Hz, 4H). | [M-H]⁻ =398.1 | Intermediates 3i and 1f. Yield: 0.05 g; 0.12 mmol; 79.4 %, brown solid. |
| | 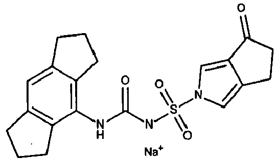 | | | |
| 1P | 1-[(3-benzoyl-1H-pyrrol-1-yl)sulfonyl]-3-(1,2,3,5,6,7-hexahydro-s-indacen-4-yl)-1-sodiourea | 1H NMR (400 MHz, DMSO-d6) δ 7.83 (s, 1H), 7.78 - 7.70 (m, 2H), 7.64-7.55 (m, 2H), 7.55 - 7.47 (m, 2H), 7.16 (dd, J = 3.0, 2.0 Hz, 1H), 6.80 (s, 1H), 6.50 (dd, J = 3.1, 1.8 Hz, 1H), 2.75 (t, J = 7.4 Hz, 4H), 2.61 (t, J = 7.3 Hz, 4H), 1.88 (p, J = 7.6 Hz, 4H). | [M-H]-=450.2 | Intermediates 3j and 1f. Yield: 0.01 g; 0.024 mmol; 6 %, white solid. |
| | 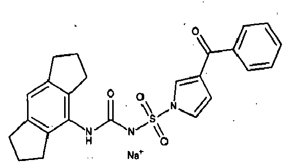 | | | |
| 1Q | 3-(1,2,3,6,7,8-hexahydroas-indacen-4-yl)-1-sodio-1-{[3-(trifluoroacetyl)-1H-pyrrol-1-yl]sulfonyl}urea | 1H NMR (400 MHz, DMSO-d6) δ 7.99 (q, J = 1.6 Hz, 1H), 7.72 (s, 1H), 7.34 (s, 1H), 7.27 (dd, J = 3.2, 1.9 Hz, 1H), 6.63 - 6.56 (m, 1H), 2.79 - 2.64 (m, 8H), 2.02 - 1.91 (m, 4H). | [M-H]⁻ =439.9 | Intermediates 3h and 11. Yield: 0.01 g, 0.02 mmol, 10 %, white solid. |
| | 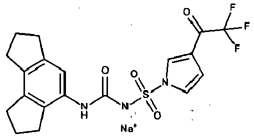 | | | |
| 1R | ammonium [(1,2,3,5,6,7-hexahydro-s-indacen-4-yl)carbamoyl][(4-oxo-4,5,6,7-tetrahydro-2H-isoindol-2-yl)sulfonyl]azanide | 1H NMR (400 MHz, DMSO-d6) δ 8.04 (s, 1H), 7.54 (d, J = 2.1 Hz, 1H), 7.21 (s, 1H), 7.00 - 6.96 (m, 1H), 2.77 (t, J = 7.4 Hz, 4H), 2.66 - 2.57 (m, 6H), 2.39 - 2.31 (m, 2H), 1.99 - 1.87 (m, 6H). | [M-H]⁻ =412.1 | Intermediates 3k and 1f. Yield: 0.04 g, 0,092 mmol, 13 %, beige solid. Compound was purified by preparative HPLC using ammonia/wate r/ACN. |
| | 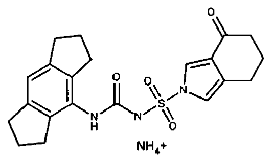 | | | |
| 1S | ammonium [(1,2,3,5,6,7-hexahydro-s-indacen-4-yl)carbamoyl][(2-methyl-1H-pyrrol-1-yl)sulfonyl]azanide | 1H NMR (400 MHz, DMSO-d6) δ 11.74 (s, 1H), 7.95 (s, 1H), 6.93 (s, 1H), 6.59 (s, 1H), 5.89 (s, 1H), 2.79 (t, J = 7.4 Hz, 4H), 2.60 (t, J = 7.3 Hz, 4H), 2.21 (s, 3H), 1.95 (p, J = 7.4 Hz, 4H). | [M-H]⁻ =358.0 | Intermediates 31 and 1f. Yield: 0.010 g, 0,026 mmol, 5 %, beige solid. Compound was purified by preparative HPLC using ammonia/wate r/ACN. |
| | 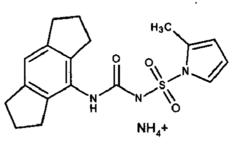 | | | |
| 1T | 3=[(3-acetyl-4-phenyl-1H-pyrrol-1-yl)sulfonyl]-1-(1,2,3,5,6,7-hexahydro-s-indacen-4-yl)-3-sodiourea propan-2-ol solvate | 1H NMR, (400 MHz, DMSO-d6) δ 7.89 (d, J = 2.3 Hz, 2H), 7.36 (d, J = 7.1 Hz, 2H), 7.29 (t, J = 7.5 Hz, 2H), 7.22 (t, J = 7.2 Hz, 1H), 7.15 (d, J = 2.2 Hz, 1H), 6.81 (s, 1 H), 4.35 (d, J = 4.0 Hz, 1H), 3.77 (d, J = 9.6 Hz, 1H), 2.76 (t, J = 7.5 Hz, 4H), 2.66 (t, J = 7.3 Hz; 4H), 2.33 (s, 3H), 1.90 (p, J = 7.4 Hz, 4H), 1.04 (d, J = 6.1 Hz, 6H). | [M+H]⁺ =464.2 | Intermediates 3m and 1f. Yield: 0.07 g, 0,128 mmol, 85 %, white solid. |
| | 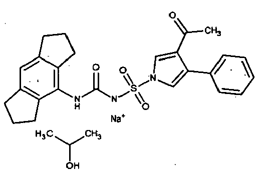 | | | |
| 1U | ammonium [(6-oxo-1,2,3,6,7,8-hexahydroas-indacen-4-yl)carbamoyl]({[3-(trifluoroacetyl)-1H-pyrrol-1-yl]sulfonyl})azanide | 1H NMR (400 MHz, DMSO-d6) δ 8.07 (s, 1H), 8.02 - 7.96 (m, 1H), 7.86 (s, 1H), 7.29 (dd, J = 3.2, 1.9 Hz, 1H), 6.64 - 6.57 (m, 1H), 2.96 - 2.79 (m, 6H), 2.62 - 2.57 (m, 2H), 2.05 (p, J = 7.8 Hz, 2H). | [M-H]-= 454.0 | Intermediates 3h and b. Yield: 30 mg, 0.08 mmol, 28 %; white solid. Compound was purified by preparative HPLC using ammonia/wate r/ACN. |
| | 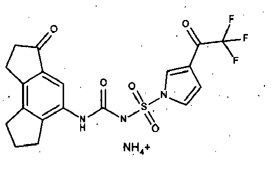 | | | |

### Example 2A

### 3-(1,2,3,5,6,7-hexahydro-s-indacen-4-yl)-1-{[3-(1-hydroxyethyl)-1H-pyrrol-1-yl]sulfonyl}urea

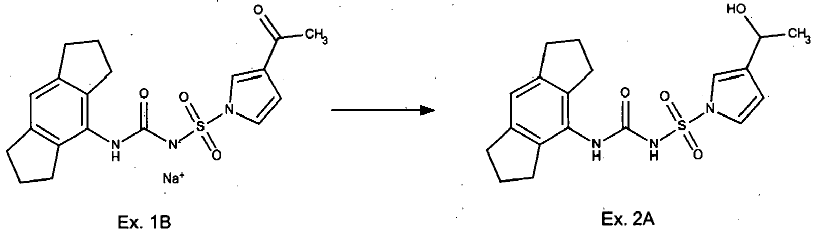

NaBH₄ (0.15 g.; 4.2 mmol) was suspended in THF (5 mL) in presence of 1-[(3-acetyl-1H-pyrrol-1-yl)sulfonyl]-3-(1,2,3,5,6,7-hexahydro-s-indacen-4-yl)-1-sodiourea (Example 1B) (0.27 g; 0.7 mmol) during a period of 15 minutes under reflux (70 °C) and stirring. Then MeOH (1.2 mL) was added dropwise with effervescence being observed. Stirring and reflux were maintained during a period of 60 minutes. Then the RM was cooled to rt and quenched with a saturated solution of NH₄Cl. Organic layer was separated and the aqueous phase extracted with EtOAc. The organic extracts were combined and dried over Na₂SO₄ and concentrated under reduced pressure. Resulted solid was treated with Et₂O, filtered and washed with 2-propanol to afford 3-(1,2,3,5,6,7-hexahydro-s-indacen-4-yl)-1-{[3-(1-hydroxyethyl)-1H-pyrrol-1-yl]sulfonyl}urea as a white solid (0.14 g, 0.359 mmol, 52 %). HPLC-MS: purity 97%, 388.1 [M-H]⁻. 1H NMR (400 MHz, DMSO-d6) δ 7.69 (s, 1H), 6.98 (t, J = 2.6 Hz, 1H), 6.95 (d, J = 2.0 Hz, 1H), 6.80 (s, 1H), 5.94 (dd, J = 2.9, 1.8 Hz, 1H), 4.66 (d, J = 4.8 Hz, 1H), 4.57 (p, J = 6.2 Hz, 1H), 2.76 (t, J = 7.4 Hz, 4H), 2.67 (t, J = 7.4 Hz, 4H), 1.91 (p, J = 7.4 Hz, 4H), 1.29 (d, J = 6.3 Hz, 3H).

The following compound was prepared by the procedure for Example 2A, using the appropriate starting materials:

| **Ex. No.** | **Name and structure** | **NMR** | **LC-MS** | **Starting materials (SM) and yield** |
|---|---|---|---|---|
| 2B | 3-(1,2,3,5,6,7-hexahydro-s-indacen-4-yl)-1-{[3-(2,2,2-trifluoro-1-hydroxyethyl)-1H-pyrrol-1-yl]sulfonyl}urea | 1H NMR (400 MHz, DMSO-d6) δ 7.74 (s, 1H), 7.21 (s, 1H), 7.07 (t, J = 2.6 Hz, 1H), 6.81 (s, 1H), 6.32 (d, J = 5.9 Hz, 1H), 6.05 (s, 1H), 4.92 (q, J = 7.1 Hz, 1H), 2.76 (t, J = 7.3 Hz, 4H), 2.65 (t, J = 7.4 Hz, 4H), 1.91 (p, J = 7.4 Hz, 4H). | [M+H]⁺ =444.2 | Example 1L. Yield: 0.032 g, 0.072 mmol, 65%, white solid. |
| | 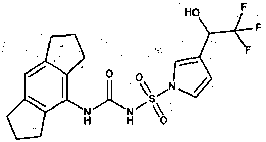 | | | |

### Example 3A

### 3-(1,2,3,5,6,7-hexahydro-s-indacen-4-yl)-1-{[3-(2-hydroxypropan-2-yl)-1H-pyrrol-1-yl]sulfonyl}-1-sodiourea

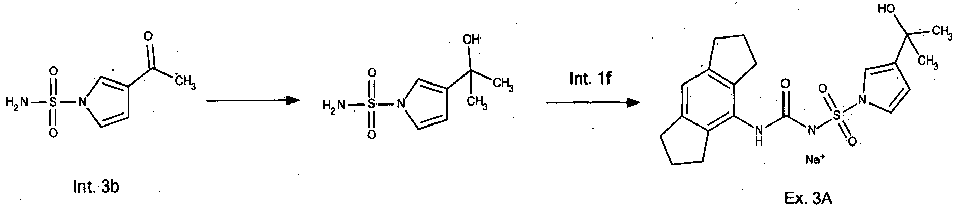

Step 1. 3-acetyl-1H-pyrrole-1-sulfonamide (Intermediate 3b) (0.3 g, 1.6 mmol) was dissolved in dry THF (15 mL) under argon. Solution was stirred at rt while methyl magnesium chloride (10.6 mL, 3M in THF) was added dropwise over 5 min. RM was stirred for overnight. The reaction was cooled to -5 °C and treated with a saturated solution of NHaCl. EtOAc was added and layers were separated. The aqueous layer was extracted second time with EtOAc. The organic extracts were combined and dried over Na₂SO₄ and concentrated under reduced pressure to afford 3-(2-hydroxypropan-2-yl)-1H-pyrrole-1-sulfonamide as a light brown oil (0.21 g, 1.02 mmol, 63 %). UPLC-MS: purity 63%, 203.1 [M-H]⁻. 1H NMR (400 MHz, DMSO-d6) δ 8.13 (s, 2H), 7.02 (dd, J = 3.1, 2.3 Hz, 1H), 6.97 (dd, J = 2.3, 1.7 Hz, 1H), 6.24 (dd, J = 3.1, 1.7 Hz, 1H), 4.80 (s, 1H), 1.37 (s, 6H). Compound was used in the next step without additional purification.

Step 2. NaH (60% in mineral oil) (0.042 g, 1.05 mmol) was added to a solution of 3-(2-hydroxypropan-2-yl)-1H-pyrrole-1-sulfonamide (0.18 g, 0.88 mmol) in anhydrous THF (1 mL). After stirring for 10 min, solution of 4-isocyanato-1,2,3,5,6,7-hexahydro-s-indacene (Intermediate If) (0.175 g, 0.88 mmol) in anhydrous THF (1 mL) was added. The RM was stirred at rt for overnight. Then solvent was evaporated at reduced pressure. The residue was treated with Et₂O, solids were filtered off and washed with Et₂O, hexane. Product was purified by crystallization from THF/Et₂O mixture to afford 3-(1,2,3,5,6,7-hexahydro-s-indacen-4-yl)-1-{[3-(2-hydroxypropan-2-yl)-1H-pyrrol-1-yl]sulfonyl}-1-sodiourea as a white solid (0.09 g, 0.211 mmol, 24 %). HPLC-MS: purity 94.2 %, 402.2 [M-H]⁻. 1H NMR (400 MHz, DMSO-d6) δ 7.67 (s, 1H), 6.96 (dd, J = 2.9, 2.2 Hz, 1H), 6.94 (t, J = 2.0 Hz, 1H), 6.79 (s, 1H), 5.96 (dd, J = 3.0, 1.8 Hz, 1H), 4.52 (s, 1H), 2.76 (t, J = 7.4 Hz, 4H), 2.67 (t, J = 7.4 Hz, 4H), 1.90 (p, J = 7.4 Hz, 4H), 1.35 (s, 6H).

### Example 4A

### 3-(1,2,3,5,6,7-hexahydro-s-indacen-4-yl)-1-{[3-(prop-1-en-2-yl)-1H-pyrrol-1-yl]sulfonyl}urea

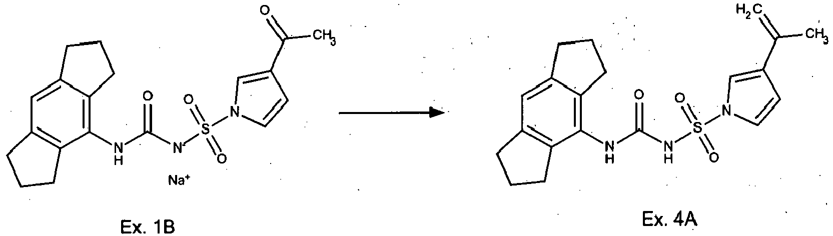

1-[(3-acetyl-1H-pyrrol-1-yl)sulfonyl]-3-(1,2,3,5,6,7-hexahydro-s-indacen-4-yl)-1-sodiourea (Example 1B) (0.1 g, 0.258 mmol) was suspended in dry THF (10 mL) under argon. Solution was stirred at rt while methyl magnesium chloride (0.86 mL, 3M in THF) was added dropwise. RM was stirred for overnight. The reaction was cooled to -5 °C and treated with a saturated solution of NH₄Cl and then acidified to pH5 with 10% solution of KHSO₄. EtOAc was added and layers were separated. The aqueous layer was extracted second time with EtOAc. The organic extracts were combined and dried over Na₂SO₄ and concentrated under reduced pressure. Product was purified by preparative TLC (EtOAc elution) to afford 3-(1,2,3,5,6,7-hexahydro-s-indacen-4-yl)-1-{[3-(prop-1-en-2-yl)-1H-pyrrol-1-yl]sulfbnyl}urea as a white solid (0.006 g, 0.014 mmol, 5.7 %). HPLC-MS: purity 90.2%, 384.2 [M-H]⁻. 1H NMR (400 MHz, DMSO-d6) δ 7.66 (s, 1H), 7.12 (t, J = 1.9 Hz, 1H), 7.01 (dd, J = 3.0,2.1 Hz, 1H), 6.79 (s, 1 H), 6.18 (dd, J = 3.0, 1.8 Hz, 1H), 5.09 (s, 1H), 4.70 (s, 1H), 2.76 (t, J = 7.3 Hz, 4H), 2.66 (t, J = 7.2 Hz, 4H), 2.01 - 1.77 (m, 7H).

### Intermediates

### 4-isocyanato-1,2,3,5,6,7-hexahydro-s-indacene (intermediate 1f)

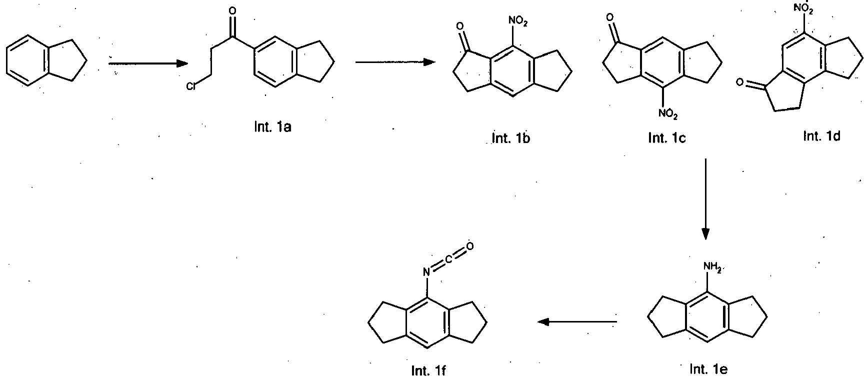

### Step 1. 3-chloro-1-(2,3-dihydro-1H-inden-5-yl)propan-1-one (Intermediate 1a)

Suspension of aluminium chloride (12.4 g, 0.093 mol) in 50 ml of DCM under a argon atmosphere was cooled to -10 °C and stirred vigorously, while a solution of 3-chloropropionyl chloride (11 g, 0.093 mol) and indan (10 g, 0.085 mol) in 15 ml of DCM was added dropwise over 0.5 h, temperature was kept between -15 °C and -5 °C. The reaction was allowed to warm to rt and stirred for overnight. The reaction mixture was added dropwise to a cold 2 M HCl over 30 min, temperature was kept between 0 °C and 10 °C. The layers were separated and the aqueous phase was washed with 30 ml of DCM three times. The combined organic layers were washed with water, saturated sodium bicarbonate, and brine. The organic phases were dried over Na₂SO₄, filtered and evaporated under reduce pressure to around 30 ml. Hexane (50 ml) was added and the evaporation continued, the procedure was repeated twice. Then after addition of 50ml, of hexane the slurry was filtered to provide 3-chloro-1-(2,3-dihydro-1H-inden-5-yl)propan-1-one as a tan solid (14.3 g, 0.068 mol, 81%). UPLC-MS: purity 99.8%. 1H NMR (400 MHz, DMSO-d6) δ 7.84 (d, 1H), 7.77 (dd, J = 7.9, 1.5 Hz, 1H), 7.37 (d, J = 7.7 Hz, 1H), 3.92 (t, J = 6.3 Hz, 2H), 3.51 (t, J = 6.3 Hz, 2H), 2.92 (t, J = 7.5 Hz, 4H), 2.05 (p, J = 7.5 Hz, 2H).

### Step 2. Mixture of 8-nitro-1,2,3,5,6,7-hexahydro-s-indacen-1-one (Intermediate 1b), 4-nitro-1,2,3,5,6,7-hexahydro-s-indacen-1-one (Intermediate 1c) and 5-nitro-1,2,3,6,7,8-hexahydroas-indacen-3-one (Intermediate 1d)

3-chloro-1-(2,3-dihydro-1H-inden-5-yl)propan-1-one (Intermediate 1a) (82 g, 0.39 mol) was added portion wise as a solid to a concentrated sulfuric acid (71 ml, 1.34 mol). The resulting mixture was heated to 60 °C for two days. Then RM was cooled to 0 °C and a mixture of nitric acid (26 ml, 0.59 mol) and sulfuric acid (26 ml, 0.49 mol) was added dropwise. RM was stirred for one hour, temperature was kept between 0 °C and 5 °C. The RM was slowly added to a mixture of water and DCM with ice bath cooling. The layers were separated and the aqueous layer was extracted with DCM. The combined organic layers were washed with brine and saturated sodium bicarbonate. The organic layers were dried over Na₂SO₄ and filtered. The crude mixture was purified by FCC (hexane/ethyl acetate). Obtained fractions was crystallized from MeOH. Obtained: 8-nitro-1,2,3,5,6,7-hexahydro-s-indacen-1-one (Intermediate 1b), (30.77g; 0.14 mol; 36 %) UPLC-MS: purity 98 %, 218 [M+H]+; 1H NMR (400 MHz, DMSO-d6) δ 7.67 (s, 1H), 3.15 - 3.08 (m, 2H), 3.04 (t, J = 7.5 Hz, 2H), 2.90 (t, J = 7.5 Hz, 2H), 2.77 - 2.71 (m, 2H), 2.13 (p, J = 7.5 Hz, 2H); 4-nitro-1,2,3,5,6,7-hexahydro-s-indacen-1-one (Intermediate 1c), (5.19 g; 0.02 mol; 5 %) UPLC-MS: purity 100 %, 218 [M+H]+; 1H NMR (400 MHz, DMSO-d6) δ 7.82 (s, 1H), 3.41 - 3.36 (m, 2H), 3.34 - 3.29 (m, 3H), 3.02 (t, J = 7.5 Hz, 2H), 2.77 - 2.69 (m, 2H), 2.13 (p, J = 7.6 Hz, 2H); 5-nitro-1,2,3,6,7,8-hexahydroas-indacen-3-one (Intermediate 1d), (3.00 g; 0.01 mol; 3.5 %) UPLC-MS: purity 100 %, 218 [M+H]+; 1H NMR (400 MHz, DMSO-d6) δ 8.09 (s, 1H), 3.39 (t, J = 7.6 Hz, 2H), 3.14 - 3.09 (m, 2H), 3:01 (t, J = 7.7 Hz, 2H), 2.81 - 2.73 (m, 2H), 2.19 (p, J = 7.7 Hz, 2H).

### Step 3. 1,2,3,5,6,7-hexahydro-s-indacen-4-amine (Intermediate 1e)

Mixture of 8-nitro-1,2,3,5,6,7-hexahydro-s-indacen-1-one (Intermediate 1b) and 4-nitro-1,2,3,5,6,7-hexahydro-s-indacen-1-one (Intermediate 1c) (7.00 g, 0.032 mol) was suspended in 70 ml of MeOH with 20% palladium hydroxide on carbon (50% water wet. 1.72 g, 0.012 mol) then methanesulfonic acid (3.41 g, 0.035 mol) was added in one portion. The mixture was hydrogenated at 35 psi for 5 h. The catalyst was removed by filtration and washed with MeOH. The filtrate was diluted with 350 ml of water and then pH was adjusted to 11 with 2 N NaOH. The resulting slurry was filtered and the crude solids were recrystallized from MeOH/water (9:1) to afford of 1,2,3,5,6,7-hexahydro-s-indacen-4-amine as a colorless, crystal needles (4.10 g, 0.023 mol, 73 %). UPLC-MS: purity 100 %, 174.1 [M+H]⁺. 1H NMR (400 MHz, DMSO-d6) δ 6.35 (s, 1H), 4.52 (s, 2H), 2.72 (t, J = 7.4 Hz, 4H), 2.59 (t, J = 7.3 Hz, 4H), 1.96 (p, J = 7.4 Hz, 4H).

### Step 4. 4-isocyanato-1,2,3,5,6,7-hexahydro-s-indacene (Intermediate If)

To a stirred solution of 1,2,3,5,6,7-hexahydro-s-indacen-4-amine (Intermediate 1e) (1.1 g, 6.35 mmol) and TEA (0.973 ml, 6.98 mmol) in 20 ml of THF, tri-phosgene (0.64 g, 2.16 mmol) was added in one portion. The mixture was heated to reflux for 4 h, then cooled to rt. THF was evaporated and the residue was taken up in pentane and filtered through a plug of silica gel. Evaporation of the pentane in vacuo afforded 4-isocyanato-1,2,3,5,6,7-hexahydro-s-indacene as a white solid (0.90 g, 46.77 mmol, 71 %). 1H NMR (400 MHz, Chloroform-d) δ 6.96 (s, 1H), 2.91 (q, J = 6.9 Hz, 8H), 2.22 - 2.03 (m, 4H).

The following compounds were prepared by the procedure for Intermediate 1e, using the appropriate starting materials:

| **Int. No.** | **Name and structure** | **NMR** | **LC-MS** | **Starting materials (SM) and yield** |
|---|---|---|---|---|
| 1g | 1H,2H,3H,5H,6H,7H,8H - cyclopenta[b]naphthalen-4-amine | 1H NMR (400 MHz, DMSO-d6) δ 6.22 (s, 1H), 4.40 (s, 2H), 2.71 (t, J = 7.4 Hz, 2H), 2.59 (q, J = 6.3, 5.5 Hz, 4H), 2.35 (t, J = 6.5 Hz, 2H), 1.93 (p, J = 7.3 Hz, 2H), 1.73 (dtt, J = 12.7, 8.6,4.8 Hz, 2H), 1.68 - 1.57 (m, 2H). | [M+H]⁺ =188.4 | 5,6,7,8-tetrahydro naphthalen -1-amine. Yield: 0.20 g, 1.06 mmol, 2%; beige solid. |
| | 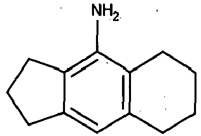 | | | |
| 1h | 1H,2H,3H,6H,7H,8H,9H-cyclopenta[a]naphthalen-5-amine | 1H NMR (400 MHz, DMSO-d6) δ 6.35 (s, 1H), 4.42 (s, 2H), 2.69 (t, J = 7.5 Hz, 2H), 2.58 (t, J = 7.3 Hz, 2H), 2.48 (t, J = 6.0 Hz, 2H), 2.32 (t, J = 6.1 Hz, 2H), 1.91 (p, J = 7.4 Hz, 2H), 1.76 - 1.60 (m, 4H). | [M+H]⁺ =188.4 | 5,6,7,8-tetrahydro naphthalen -1-amine. Yield: 0.15 g, 0.8 mmol, 1%; brown oil. |
| | 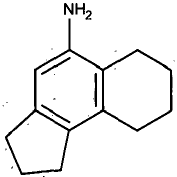 | | | |
| 1i | 1,2,3,6,7,8-hexahydroas-indacen-4-amine | 1H NMR (400 MHz, DMSO-d6) δ 6.28 (s, 1H), 4.51 (s, 2H), 2.68 (q, J = 7.0 Hz, 4H), 2.60 (q, J = 6.9 Hz, 4H), 1.95 (dp, J = 14.6, 7.4 Hz, 4H). | [M+H]⁺ =174.4 | Intermedia te 1d, hydrogena tion during 24h. Yield: 0.18 g, 1.03 mmol, 45%; brown oil. |
| | 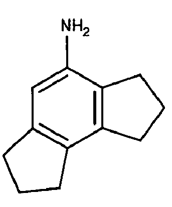 | | | |

The following compounds were prepared by the procedure for Intermediate 1f, using the appropriate starting materials:

| **Int. No.** | **Name and structure** | **Starting materials (SM) and yield** |
|---|---|---|
| 1j | 4-isocyanato-1H,2H,3H,5H,6H,7H,8H-cyclopenta[b]naphthalene | Intermediate 1g. Yield: 0.1 g, 0.47 mmol, 45%; brown oil. |
| | 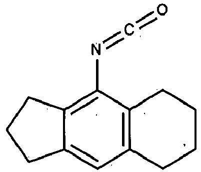 | |
| 1k | 5-isocyanato-1H,2H,3H,6H,7H,8H,9H-cyclopenta[a]naphthalene | Intermediates 1h. Yield: 0.1 g, 0.47 mmol, 55%; brown oil. |
| | 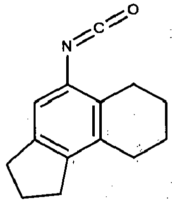 | |
| 11 | 4-isocyanato-1,2,3,6,7,8-hexahydroas-indacene | Intermediate 1i. Yield: 0.1 g, 0.50 mmol, 55%; brown oil. |
| | 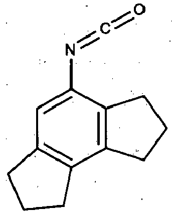 | |

### Intermediate 2a

### tert-butyl N-(chlorosulfonyl)carbamate

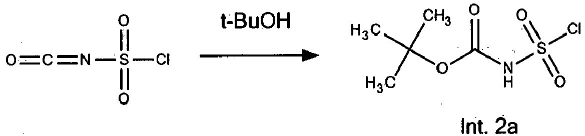

t-BuOH (6.04 mL, 63 mmol) was added dropwise to a solution of chlorosulfonyl isocyanate (4.99 mL, 57 mmol) in anhydrous DCM (30 mL) under argon at -30 °C and stirred at 0 °C for 40 min. Then the resulting RM was concentrated in vacuo, residue was treated with hexane and filtered, dried in vacuo resulting the product as a white solid (8 g, 37 mmol), 64.5 %).

### Intermediate 3a

### 1H-indole-1-sulfonamide

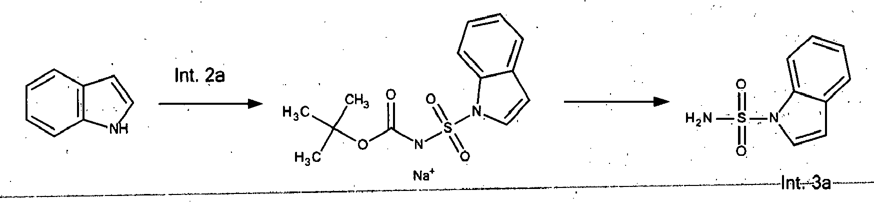

Step 1. NaH (60% in mineral oil) (0.408 g, 10.2 mmol) was added to a solution of 1H-indole (0.598 g, 5.1 mmol) in anhydrous THF (6 mL) at 0 °C. The resulting RM was stirred at 0 °C for 30 min. Then a solution of tert-butyl N-(chlorosulfonyl)carbamate (Intermediate 2a) (1 g, 4.64 mmol) in anhydrous THF (8 mL) was added. The RM was stirred at rt for overnight. Then solvent was evaporated and the residue was treated with hexane and filtered off to afford tert-butyl N-(1H-indole-1-sulfonyl)-N-sodiocarbamate as a grey solid (1.47 g, 4.64 mmol, 100%). UPLC-MS: purity 79 %, 295 [M-H]⁻. The compound was used in the next step without additional purification.

Step 2. TFA (7.1 mL, 92.7 mmol) was added to a solution of tert-butyl N-(1H-indole-1-sulfonyl)-N-sodiocarbamate (1.476 g, 4.64 mmol) in anhydrous DCM (20 mL). The resulting solution was stirred overnight at rt. Then the mixture was concentrated in vacuo. Product was purified by FCC on silica gel (MeOH gradient in DCM) to afford 1H-indole-1-sulfonamide (Intermediate 3a) as a beige solid (0.182 g, 0.93 mmol, 20% yield). UPLC-MS: purity 100 %, 195.15 [M-H]⁻. 1H NMR (400 MHz, DMSO-d6) δ 8.31 (s, 2H), 7.87 (dt, J = 8.3, 0.9 Hz, 1H), 7.65 (dt, J = 7.8, 1.0 Hz, 1H), 7.55 (d, J = 3.6 Hz, 1H), 7.32 (ddd, J = 8.4, 7.2, 1.3 Hz, 1H), 7.24 (ddd, J = 8.1, 7.2, 1.0 Hz, 1H), 6.70 (dd, J = 3.6, 0.8 Hz, 1H).

The following compounds were prepared by the procedure for Intermediate 3a, using the appropriate starting materials:

| **Int. No.** | **Name and structure** | **NMR** | **LC-MS** | **Starting materials (SM) and yield** |
|---|---|---|---|---|
| 3b | 3-acetyl-1H-pyrrole-1-sulfonamide | 1H NMR (400 MHz, DMSO-d6) δ 8.57 (s, 2H), 7.89 (t, J = 1.9 Hz, 1H), 7.21 (dd, J = 3.2, 2.2 Hz, 1H), 6.62 (dd, J = 3.2, 1.7 Hz, 1H), 2.39 (s, 3H). | [M-H]⁻ =186.9 | 1-(1H-pyrrol-3-yl)ethan-1-one. Yield: 0.45 g, 2.4 mmol, 22%; beige solid. |
| | 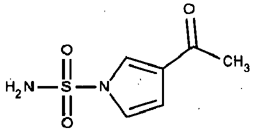 | | | |
| 3c | methyl 1-sulfamoyl-1H-pyrrole-3-carboxylate | 1H NMR (400 MHz, DMSO-d6) δ 8.59 (s, 2H), 7.76 - 7.64 (m, 1H), 7.22 (dd, J = 3.2, 2.3 Hz, 1H), 6.61 (dd, J = 3.2, 1.7 Hz, 1H), 3.76 (s, 3H). | [M-H]⁻ =202.9 | Methyl 1H-pyrrole-3-carboxylate. Yield: 1 g, 4.9 mmol, 17%; light green solid. |
| | 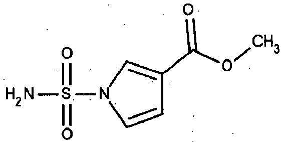 | | | |
| 3d | 1H-pyrrolo[3,2-b]pyridine-1-sulfonamide | 1H NMR (400 MHz, DMSO-d6) δ 8.71 - 8.59 (m, 3H), 8.38 (dt, J = 8.5, 1.1 Hz, 1H), 8.00 (d, J = 3.7 Hz, 1H), 7.52 (dd, J = 8.4, 4.9 Hz, 1H), 6.91 (dd, J = 3.7, 0.8 Hz, 1H). | [M-H]⁻ =196.1 | 1H-pyrrolo[3,2-b]pyridine. Yield: 0.15 g, 0.77 mmol, 23%; beige solid. |
| | 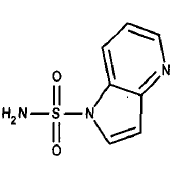 | | | |
| 3e | 1H-pyrrolo[2,3-c]pyridine-1-sulfonamide | | [M+H]⁺ =198.3 | 1H-pyrrolo[2,3-c]pyridine. Yield: 0.08 g, 0.41 mmol, 20%; beige solid. |
| | 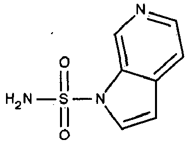 | | | |
| 3f | 3-nitro-1H-pyrrole-1-sulfonamide | 1H NMR (400 MHz, DMSO-d6) δ 8.90 (s, 2H), 8.13 (dd, J = 2.5, 1.8 Hz, 1H), 7.30 (dd, J = 3.5, 2.5 Hz, 1H), 6.90 (dd, J = 3.5, 1.8 Hz, 1H). | [M-H]⁻ =190.1 | 3-nitro-1H-pyrrole. Yield: 0.06 g, 0.329 mmol, 45%; beige solid. |
| | 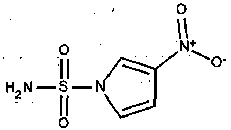 | | | |
| 3g | 1H-pyrrole-1-sulfonamide | 1H NMR (400 MHz, DMSO-d6) δ 8.21 (s, 2H), 7.16 - 713 (m, 2H), 6.29-6.24 (m, 2H). | [M+H]⁺ =146.4 | 1H-pyrrole. Yield: 0.06 g, 0.397 mmol, 19%; beige solid. |
| | 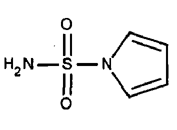 | | | |
| 3h | 3-(trifluoroacetyl)-1H-pyrrole-1-sulfonamide | 1H NMR (400 MHz, DMSO-d6) δ 8.89 (s, 2H), 8.02 (p, J = 1.6 Hz, 1H), 7.41 (dd, J = 3.3, 2.1 Hz, 1H),6.84 (dd, J = 3.3, 1.7 Hz, 1H). | [M-H]⁻ =241.0 | 2,2,2-trifluoro-1-(1H-pyrrol-3-yl)ethan-1-one. Yield: 0.25 g, 1.03 mmol, 22%; white solid. |
| | 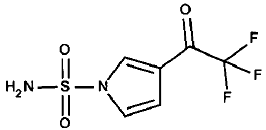 | | 5 | |
| 3i | 4-oxo-2H,4H,5H,6H-cyclopenta[c]pyrrole-2-sulfonamide | | [M-H]⁻ =199.1 | 2H,4H,5H,6H-cyclopenta[c]pyr rol-4-one. Yield: 0.03 g, 0.15 mmol, 11%; white solid. |
| | 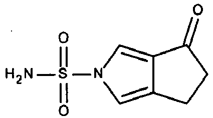 | | | |
| 3j | 3-benzoyl-1H-pyrrole-1-sulfonamide | | [M-H]⁻ =249.1 | 3-benzoyl-1H-pyrrole. Yield: 0.05 g, 0.2 mmol, 7.15 %; beige solid. |
| | 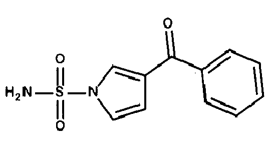 | | | |
| 3k | 4-oxo-4,5,6,7-tetrahydro-2H-isoindole-2-sulfonamide | | [M-H]⁻ =213.1 | 4,5,6,7-tetrahydro-2H-isoindol-4-one. Yield: 0.75g, 3.50 mmol, 53%, yellow solid. |
| | 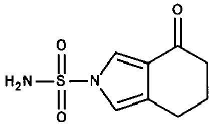 | | | |
| 31 | 2-methyl-1H-pyrrole-1-sulfonamide | | [M-H]⁻ =159.2 | 2-methyl-1H-pyrrole. Yield: 0.23g, 1.46 mmol, 19%, white solid. |
| | 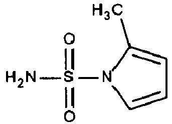 | | | |
| 3m | 3-acetyl-4-phenyl-1H-pyrrole-1-sulfonamide | 1H NMR (400 MHz, DMSO-d6) δ 11.55 (s, 1H), 7.70 (dd, J = 3.2, 2.1 Hz, 1H), 7.44 - 7.37 (m, 2H), 7.31 - 7.26 (m, 2H), 7.22 - 7.17 (m, 1H), 6.93 (t, J = 2.3 Hz, 1H), 2.33 (s, 3H). | [M-H]⁻ =184.2 | 1-(4-phenyl-1H-pyrrol-3-yl)ethan-1-one. Yield: 0.32g, 1.75 mmol, 85.4 %, light yellow solid. |
| | 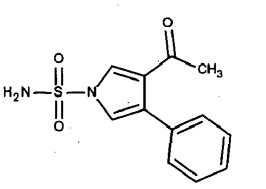 | | | |

### Intermediate 4a

### 1H-indazole-1-sulfonamide

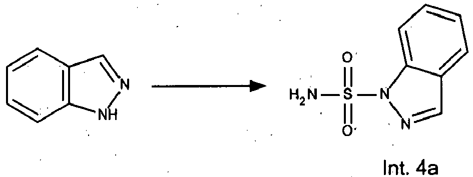

Sulfamoyl chloride (0.64 g, 5.58 mmol) was added to a solution of 1H-indazole in DMA at 0 °C. The mixture was stirred at 0 °C for 1h and then left at rt for overnight. Then the RM was treated with cooled brine, extracted with EtOAc and combined organic extracts were washed with water, brine and dried over Na₂SO₄. Solvent was removed under reduced pressure and obtained oily residue was treated with water, separated solids were filtered and washed with water to afford 1H-indazole-1-sulfonamide as a beige solid (0.26 g, 1.32 mmol, 52% yield). UPLC-MS: purity 99 %, 196.1 [M-H]⁻. 1H NMR (400 MHz, DMSO-d6) δ 8.56 (s, 2H), 8.46 (d, J = 0.8 Hz, 1H), 8.01 - 7.93 (m, 1H), 7.89 (dd, J = 8.0, 1.1 Hz, 1H), 7.59 (ddd, J = 8.4, 7.0, 1.2 Hz, 1H), 7.42 - 7.30 (m, 1H).

### Intermediates 5a and 5b

### 3-cyclopropyl-1H-pyrrole-1-sulfonamide (Intermediate 5a) and 3-(1-hydroxycyclopropyl)-1H-pyrrole-1-sulfonamide (Intermediate 5b)

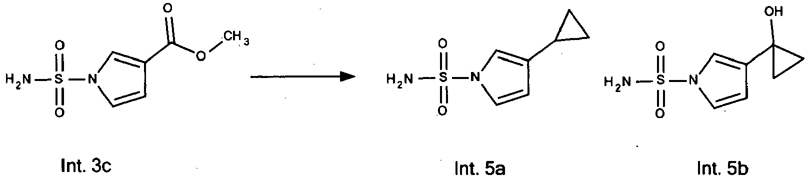

To a solution of methyl 1-sulfamoyl-1H-pyrrole-3-carboxylate (Intermediate 3c) (0.5 g, 2.45 mmol) in anhydrous THF was added TTIP (2.17 mL, 7.34 mmol). To the stirring mixture ethylmagnesium bromide (24.5 mL, 1M in THF) was slowly added. The RM was stirred at RT for overnight. The reaction was quenched with saturated solution of NH₄Cl and extracted with EtOAc. Organic layer was separated, washed with brine, dried over Na₂SO₄ and purified by FCC (MeOH gradient in DCM) to afford the title compounds. Intermediate 5a: 0.03 g, 0.16 mmol, 6.6 % yield as a brown solid. UPLC-MS: purity 62 %, 185.0 [M-H]⁻. 1H NMR (400 MHz, DMSO-d6) δ 8.07 (s, 2H), 6.99 (dd, J = 3.1, 2.3 Hz, 1H), 6.91 (ddd, J = 2.3, 1.7, 0.6 Hz, 1H), 6.02 (dd, J = 3.1, 1.7 Hz, 1H), 1.74 - 1.69 (m, 1H), 0.82 - 0.76 (m, 2H), 0.50 *-* 0.44 (m, 2H). Intermediate 5b: 0.06 g, 0.29 mmol, 12.2 % yield as a white solid. UPLC-MS: purity 85 %, 201.1 [M-H]⁻. 1H NMR (400 MHz, DMSO-d6) δ 8.12 (s, 2H), 7.01 (t, J = 2.7 Hz, 1H), 6.98 (t, J = 2.0 Hz, 1H), 5.97 (dd, J = 3.1, 1.7 Hz, 1H), 5.79 (s, 1H), 0.95 (q, J = 4.6 Hz, 2H), 0.73 (q, J = 4.6 Hz, 2H).

### Intermediate 6b

### 4-chloro-8-isocyanato-1,2,3,5,6,7-hexahydro-s-indacene

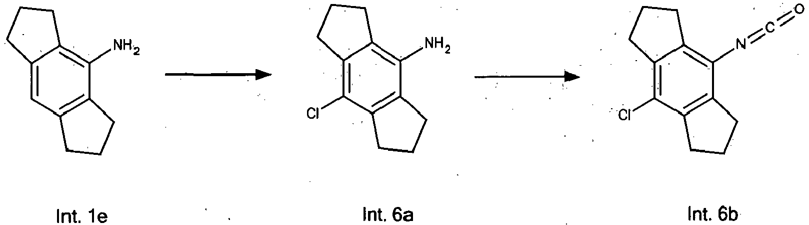

### Step 1. 8-chloro-1,2,3,5,6,7-hexahydro-s-indacen-4-amine (Intermediate 6a)

To a solution of 1,2,3,5,6,7-hexahydro-s-indacen-4-amine (Intermediate 1e) (0.35 g, 2.02 mmol) in acetonitrile (10 ml) was added N-chlorosuccinimide (0.29 g, 2.17 mmol) and the resulting RM was stirred at rt for overnight. Then solvent was removed in vacuo and the residue was purified by FCC (hexane/EtOAc elution) to afford 8-chloro-1,2,3,5,6,7-hexahydro-s-indacen-4-amine (Intermediate 7a) as a beige solid (0.40 g, 1.92 mmol, 95 % yield). UPLC-MS: purity 95 %, 208.3 [M+H]⁺.

Step 2. Compound was prepared by the procedure for Intermediate 1f affording 4-chloro-8-isocyanato-1,2,3,5,6,7-hexahydro-s-indacene as a white solid (0.28 g, 1.19 mmol, 62 %).

### Intermediate 7b

### 5-isocyanato-1,2,3,6,7,8-hexahydroas-indacen-3-one

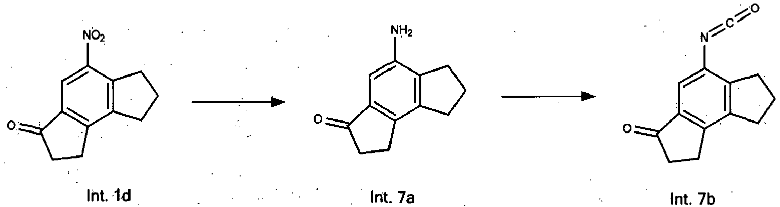

### Step 1. 5-amino-1,2,3,6,7,8-hexahydroas-indacen-3-one (Intermediate 7a)

5-nitro-1,2,3,6,7,8-hexahydroas-indacen-3-one (Intermediate 1d) (1.5 g; 6.91 mmol;) was suspended in 15 ml of MeOH with 20% palladium hydroxide on carbon (50% water wet. 197 mg, 0.69 mmol) then methanesulfonic acid (0.73 g, 0.49 ml, 7.59 mmol) was added in one portion. The mixture was hydrogenated at 35 psi for 5 h. The catalyst was removed by filtration and washed with MeOH. The filtrate was diluted with 350 ml of water and then pH was adjusted to 11 with 2 N NaOH. RM was concentrated and extracted with DCM 3 times. Crude product was purified by FCC to afford 5-amino-1,2,3,6,7,8-hexahydroas-indacen-3-one (0.25 g, 1.33 mmol, 19 %). UPLC-MS: purity 93 %, 188.35 [M+H]⁺. 1H NMR (400 MHz, DMSO-d6) δ 6.65 (s, 1H), 5.05 (s, 2H), 2.86 - 2.78 (m, 4H), 2.72 (t, J = 7.5 Hz, 2H), 2.55 - 2.50 (m, 2H), 2.07 (p, J = 7.6 Hz, 2H).

Step 2. Compound was prepared by the procedure for Intermediate 1f affording 5-isocyanato-1,2,3,6,7,8-hexahydroas-indacen-3-one as a brown solid (0.20 g, 0.95 mmol, 89 %). 1H NMR (400 MHz, Chloroform-d) δ 7.30 (s, 1H), 3.10 - 2.94 (m, 6H), 2.78 - 2.69 (m, 2H), 2.25 (p, J = 7.6 Hz, 2H).

### 3.1.Determination of the inhibitory activity in vitro

The biological activity of the compounds of the present invention was determined utilizing the assay described hereinafter.

### PBMC IC50 determination assay

The compounds of the present invention were tested for their inhibitory activity against IL1-β release upon NLRP3 activation in peripheral blood mononuclear cells (PBMC).

PBMC were isolated from buffy coats by density gradient centrifugation on Histopaque-1077 (Sigma, cat no. 10771). Isolated cells were seeded into the wells of a 96-well plate and incubated for 3 h with lipopolysaccharide (LPS). Following medium exchange, the compounds of the present invention were added (a single compound per well) and the cells were incubated for 30 min. Next, the cells were stimulated with nigericin (10 µM) for 1 h and the cell culture media from the wells were collected for further analysis.

The release of IL1-β into the media was determined by a quantitative detection of IL1-β in the media using an IL-1β enzyme-linked immunosorbent assay (ELISA) Ready-SET-Go!, eBioscience cat. No. 88-7261-88. Briefly, in a first step, high affinity binding plates (Coming, Costar 9018 or NUNC Maxisorp Cat No. 44-2404) were coated overnight at 4°C with specific capture antibody included in the kit (anti-human IL-1β ref. 14-7018-68). Subsequently, plates were blocked with blocking buffer for 1 h at room temperature (rt) and after washing with a buffer (PBS with 0,05% Tween-20) incubated with protein standard and culture media. After 2 h of incubation at rt, plates were washed and incubated with biotinylated detection antibody included in the kit (anti-human IL-1β Biotin ref. 33-7110-68) for 1 h at rt. Plates were washed and incubated with HRP-streptavidin for 30 min at rt and washed again. The signal was developed after addition of 3,39,5,59-tetramethylbenzidine-peroxidase (TMB) until color appeared and the reaction was stopped by 2 N H₂SO₄. A microplate spectrophotometer (BioTek) was used to detect signals with 450 nM. The detection range of IL1-β ELISA was 2-150 ng/ml.

The determination of the IC₅₀ values were preformed using the Graph Pad Prism software and the measured IC₅₀ values of compounds of the present invention are shown in Table 1 below.

**Table 1**

| **Example No.** | **PBMCs, IC₅₀, µM** |
|---|---|
| 1K | 1.40 |
| 1C | 4.07* |
| 3A | 0.66* |
| 1B | 1.27* |
| 2A | 1.97 |
| 4A | 0.81* |
| 1L | 0.074* |
| 1A | 1.05 |
| 1D | 10.5 |
| 1E | 1.0 |
| 1F | 0.4 |
| 1I | 0.91 |
| 1H | 4.93 |
| 1J | 26.8 |
| 1G | 5.0 |
| 1M | 4.62 |
| 1N | 2.03 |
| 1O | 4.13 |
| 1P | 2.91 |
| 2B | 0.98 |
| 1Q | 0.33 |
| 1U | 1.82 |
| 1S | 0.27* |
| 1T | 4.76 |
| 1R | 1.27 |

| | |
|---|---|
| *mean values based on 2-6 measurements. All other values quoted in above table are single measurements. | |

These results show that the compounds of the present invention are capable of inhibiting IL1-β release upon inflammasome activation.

## Claims

1. A compound of Formula (I): 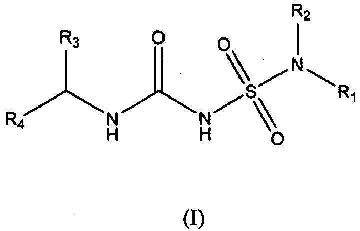 in which:
R₁ and R₂, together with the nitrogen atom to which they are attached to, form a 5 membered monocyclic heteroaryl ring system, wherein the monocyclic heteroaryl ring system, in addition to the nitrogen atom to which R₁ and R₂ are attached to, optionally comprises 1, 2 or 3 further heteroatoms independently selected from oxygen, nitrogen and sulfur; or
R₁ and R₂, together with the nitrogen atom to which they are attached to, form a 8, 9 or 10 membered bicyclic heteroaryl ring system, wherein the bicyclic heteroaryl ring system, in addition to the nitrogen atom to which R₁ and R₂ are attached to, optionally comprises 1, 2 or 3 further heteroatoms independently selected from oxygen, nitrogen and sulfur;
wherein said monocyclic heteroaryl ring system or said bicyclic heteroaryl ring system is optionally substituted by 1, 2, or 3 substituents independently selected from (1-6C)alkyl, (2-6C)alkenylene, (2-6C)alkynylene, (3-8C)cycloalkyl, halo, nitro, cyano, CF₃, oxo, OR₅, N(R₆)(R₇), NR₅C(O)R₆, NR₆S(O)₂R₈, N(R₅)C(O)N(R₆)(R₇), S(O)₂R₈, S(O)R₈, S(O)(NR₅)(R₈), S(O)₂N(R₆)(R₇), C(O)OR₅, C(O)N(R₆)(R₇), C(O)R₈, C(NOR₅)(R₈), OC(O)N(R₆)(R₇), OC(O)R₈, phenyl, a 5 or 6 membered monocyclic heteroaryl ring system comprising 1, 2 or 3 heteroatoms independently selected from oxygen, nitrogen and sulfur, and a 3, 4, 5, or 6 membered monocyclic heterocyclyl ring system comprising 1 or 2 heteroatoms independently selected from oxygen, nitrogen and sulfur, wherein said (1-6C)alkyl, (2-6C)alkenylene, (2-6C)alkynylene, (3-8C)cycloalkyl, phenyl, 5 or 6 membered monocyclic heteroaryl ring system or 3, 4, 5, or 6 membered monocyclic heterocyclyl ring system is optionally substituted by 1, 2, 3 or 4 substituents independently selected from halo, cyano, oxo, CF₃, OR₅, N(R₆)(R₇), NR₅C(O)R₆, SR₅, C(O)N(R₆)(R₇), S(O)₂R₈, SOR₈, S(O)₂N(R₅)(R₆) and NR₅S(O)₂R₆;
R₃ and R₄, together with the carbon atom to which they are attached to, form a 12, 13, 14, 15 or 16 membered tricyclic partially unsaturated heterocyclic or carbocyclic ring system, wherein said tricyclic ring system is optionally substituted by 1, 2, 3 or 4 substituents independently selected from (1-6C)alkyl, (2-6C)alkenylene, (2-6C)alkynylene, (3-8C)cycloalkyl, (1-3C)alkoxy, halo, oxo, hydroxy, cyano, amino, (1-3C)alkylamino, di-[(1-3C)alkyl]-amino, CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃;
R₅, R₆ and R₇ are each independently selected from H, (1-6C)alkyl, CF₃, phenyl, a 5 or 6 membered monocyclic heteroaryl ring system comprising 1, 2 or 3 heteroatoms independently selected from oxygen, nitrogen and sulfur, a 3, 4, 5 or 6 membered monocyclic heterocyclyl ring system comprising 1 or 2 heteroatoms independently selected from oxygen, nitrogen and sulfur and a 3, 4, 5 or 6 membered saturated or partially unsaturated carbocyclic ring system, wherein said (1-6C)alkyl, phenyl, monocyclic heteroaryl ring, monocyclic heterocyclyl ring or carbocyclic ring system is optionally substituted with 1 or 2 substituents independently selected from halo, amino, methylamino, di(methyl)amino, nitro, hydroxy, methoxy, oxo, cyano, C(O)NH₂, C(O)NHCH₃, C(O)N(CH₃)(CH₃), CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃;
each R₈ is independently selected from (1-6C)alkyl, CF₃, phenyl, a 5 or 6 membered monocyclic heteroaryl ring system comprising 1, 2 or 3 heteroatoms independently selected from oxygen, nitrogen and sulfur, a 3, 4, 5 or 6 membered monocyclic heterocyclyl ring system comprising 1 or 2 heteroatoms independently selected from oxygen, nitrogen and sulfur and a 3, 4, 5 or 6 membered saturated or partially unsaturated carbocyclic ring system, wherein said (1-6C)alkyl, phenyl, monocyclic heteroaryl ring, monocyclic heterocyclyl ring or carbocyclic ring system is optionally substituted with 1 or 2 substituents independently selected from halo, amino, methylamino, di(methyl)amino, nitro, hydroxy, methoxy, oxo, cyano, C(O)NH₂, C(O)NHCH₃, C(O)N(CH₃)(CH₃), CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃; or a pharmaceutically acceptable salt thereof.

2. A compound, or pharmaceutically acceptable salt thereof, according to claim 1, wherein R₁ and R₂, together with the nitrogen atom to which they are attached to, form a 5 membered monocyclic heteroaryl ring system selected from pyrrolyl, isoxazolyl, thiazolyl, isothiazolyl, pyrazolyl, imidazolyl, oxadiazolyl, thiadiazolyl, triazolyl and tetrazolyl; or R₁ and R₂, together with the nitrogen atom to which they are attached to, form a 8, 9 or 10 membered bicyclic heteroaryl ring system selected from indolyl, isoindolyl, indazolyl, 4,5,6,7-tetrahydroindolyl, 4,5,6,7-tetrahydroisoindolyl, benzimidazolyl, benzoxathiazolyl, benzoxadiazolyl, benzothiadiazolyl, quinolinyl, isoquinolinyl, purinyl, 1,8-naphthyridyl, pteridyl, 1H-pyrrolo[3,2-b]pyridinyl, 1H-pyrrolo[2,3-c]pyridinyl, 2H,4H,5H,6H-cyclopenta[c]pyrrolyl, pyrido[3,2-d]pyrimidyl and pyridoimidazolyl.

3. A compound, or pharmaceutically acceptable salt thereof, according to claim 1, wherein R₁ and R₂, together with the nitrogen atom to which they are attached to, form a ring system selected from pyrrolyl, indolyl, indazolyl, 1H-pyrrolo[3,2-b]pyridinyl, 1H-pyrrolo[2,3-c]pyridinyl and 2H,4H,5H,6H-cyclopenta[c]pyrrolyl.

4. A compound, or pharmaceutically acceptable salt thereof, according to claim 1, wherein R₁ and R₂, together with the nitrogen atom to which they are attached to, form a ring system selected from pyrrolyl, indolyl, indazolyl, 4,5,6,7-tetrahydroisoindolyl, 1H-pyrrolo[3,2-b]pyridinyl, 1H-pyrrolo[2,3-c]pyridinyl and 2H,4H,5H,6H-cyclopenta[c]pyrrolyl, wherein said ring system is optionally substituted by 1, 2, or 3 substituents independently selected from (1-6C)alkyl, (2-6C)alkenylene, (2-6C)alkynylene, (3-8C)cycloalkyl, halo, cyano, CF₃, oxo, amino, (1-3C)alkylamino, di-[(1-3C)alkyl]-amino, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃, OR₅, C(O)OR₅, C(O)R₈ and phenyl, wherein said (1-6C)alkyl, (2-6C)alkenylene, (2-6C)alkynylene, (3-8C)cycloalkyl or phenyl is optionally substituted by 1, 2, 3 or 4 substituents independently selected from halo, CF₃ and hydroxy, wherein each R₈ is independently selected from (1-6C)alkyl, (1-3C)alkoxy and CF₃, and wherein each R₅ is (1-6C)alkyl;

5. A compound, or pharmaceutically acceptable salt thereof, according to claim 1, wherein R₁ and R₂, together with the nitrogen atom to which they are attached to, form a ring system selected from pyrrolyl, indolyl, indazolyl, 4,5,6,7-tetrahydroisoindolyl, 1H-pyrrolo[3,2-b]pyridinyl, 1H-pyrrolo[2,3-c]pyridinyl and 2H,4H,5H,6H-cyclopenta[c]pyrrolyl, wherein said ring system is optionally substituted by 1, 2, or 3 substituents independently selected from (1-6C)alkyl, (2-6C)alkenylene, (3-8C)cycloalkyl, oxo, C(O)OR₅, C(O)R₈ and phenyl, wherein said (1-6C)alkyl is optionally substituted by 1, 2, 3 or 4 substituents independently selected from halo, CF₃ and hydroxy, wherein each R₈ is independently selected from (1-6C)alkyl, (1-3C)alkoxy and CF₃, and wherein each R₅ is (1-6C)alkyl.

6. A compound, or pharmaceutically acceptable salt thereof, according to claim 1, wherein R₁ and R₂, together with the nitrogen atom to which they are attached to, form a pyrrolyl ring, which is optionally substituted by 1, 2, or 3 substituents independently selected from (1-6C)alkyl, (2-6C)alkenylene, (3-8C)cycloalkyl, oxo, C(O)OR₅, C(O)R₈ and phenyl, wherein said (1-6C)alkyl is optionally substituted by 1, 2, 3 or 4 substituents independently selected from halo, CF₃ and hydroxy, wherein each R₈ is independently selected from (1-6C)alkyl, (1-3C)alkoxy and CF₃, and wherein each R₅ is (1-6C)alkyl.

7. A compound, or pharmaceutically acceptable salt thereof, according to any one of claims 1 to 6, wherein R₃ and R₄, together with the carbon atom to which they are attached to, form a 12, 13, 14, 15 or 16 membered tricyclic partially unsaturated heterocyclic or carbocyclic ring system, wherein said tricyclic ring system is optionally substituted by 1 or 2 substituents independently selected from (1-6C)alkyl, (2-6C)alkenylene, (2-6C)alkynylene, (3-8C)cycloalkyl, halo, oxo, hydroxy, cyano, amino, (1-3C)alkylamino, di-[(1-3C)alkyl]-amino, CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃.

8. A compound, or pharmaceutically acceptable salt thereof, according to any one of claims 1 to 6, wherein R₃ and R₄, together with the carbon atom to which they are attached to, form a 12, 13, 14, 15 or 16 membered tricyclic partially unsaturated heterocyclic or carbocyclic ring system, wherein said tricyclic ring system is optionally substituted by 1 or 2 substituents independently selected from (1-6C)alkyl, halo and oxo.

9. A compound, or pharmaceutically acceptable salt thereof, according to any one of claims 1 to 6, wherein R₃ and R₄, together with the carbon atom to which they are attached to, form a 12, 13, 14, 15 or 16 membered tricyclic partially unsaturated carbocyclic ring system selected from the group consisting of 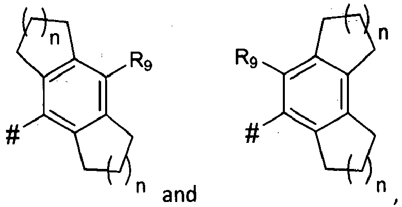 wherein # denotes the bond to the nitrogen atom of Formula (I); wherein n is an integer independently selected from 1, 2 and 3; and wherein R₉ is selected from the group consisting of hydrogen, (1-6C)alkyl, (2-6C)alkenylene, (2-6C)alkynylene, (3-8C)cycloalkyl, halo, oxo, hydroxy, cyano, amino, (1-3C)alkylamino, di-[(1-3C)alkyl]-amino, CF₃, OCF₃, S(O)₂CH₃, S(O)CH₃, S(O)₂NH₂, S(O)₂NHCH₃, S(O)₂N(CH₃)₂, NHS(O)₂CH₃ and N(CH3)S(O)₂CH₃.

10. A compound, or pharmaceutically acceptable salt thereof, according to any one of claims 1 to 6, wherein R₃ and R₄, together with the carbon atom to which they are attached to, form a 12, 13, 14, 15 or 16 membered tricyclic partially unsaturated carbocyclic ring system selected from the group consisting of 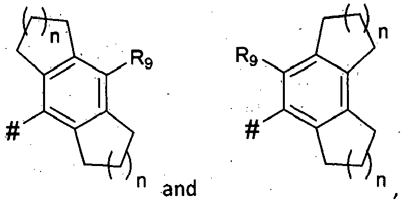 wherein # denotes the bond to the nitrogen atom of Formula (I); wherein n is an integer independently selected from 1, 2 and 3; and wherein R₉ is selected from the group consisting of hydrogen, (1-6C)alkyl, halo, CF₃ and OCF₃.

11. A compound, or pharmaceutically acceptable salt thereof, according to any one of claims 1 to 6, wherein R₃ and R₄, together with the carbon atom to which they are attached to, form a hexahydroindacene selected from the group consisting of 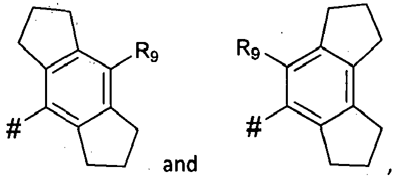 wherein # denotes the bond to the nitrogen atom of Formula (I); and wherein R₉ is selected from the group consisting of hydrogen, (1-6C)alkyl, (2-6C)alkenylene, (2-6C)alkynylene, (3-8C)cycloalkyl, halo, oxo, hydroxy, cyano, amino, (1-3C)alkylamino, di-[(1-3C)alkyl]-amino, CF₃, OCF₃, S(O)₂CH₃, S(O)CH₃, S(O)₂NH₂, S(O)₂NHCH₃, S(O)₂N(CH₃)₂, NHS(O)₂CH₃ and N(CH₃)S(O)₂CH₃.

12. A compound, or pharmaceutically acceptable salt thereof, according to any one of claims 1 to 6, wherein R₃ and R₄, together with the carbon atom to which they are attached to, form a hexahydroindacene selected from the group consisting of 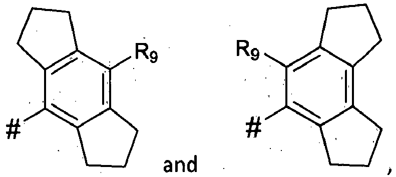 wherein # denotes the bond to the nitrogen atom of Formula (I); and wherein R₉ is selected from hydrogen, (1-6C)alkyl, halo, CF₃ and OCF₃;

13. A compound, or pharmaceutically acceptable salt thereof, according to claim 1, which is selected from any one of the following:
3-(1,2,3,5,6,7-hexahydro-s-indacen-4-yl)-1-(1H-pyrrole-1-sulfonyl)urea;
methyl1-({[(1,2,3,5,6,7-hexahydro-s-indacen-4-yl)carbamoyl]amino}sulfonyl)-1H-pyrrole-3-carboxylate;
3-(1,2,3,5,6,7-hexahydro-s-indacen-4-yl)-1-{[3-(2-hydroxypropan-2-yl)-1H-pyrrol-1-yl]sulfonyl} urea;
1-[(3-acetyl-1H-pyrrol-1-yl)sulfonyl]-3-(1,2,3,5,6,7-hexahydro-s-indacen-4-yl)urea;
3-(1,2,3,5,6,7-hexahydro-s-indacen-4-yl)-1-{[3-(1-hydroxyethyl)-1H-pyrrol-1-yl]sulfonyl}urea;
3-(1,2,3,5,6,7-hexahydro-s-indacen-4-yl)-1-{[3-(prop-1-en-2-yl)-1H-pyrrol-1-yl]sulfonyl}urea;
3-(1,2,3,5,6,7-hexahydro-s-indacen-4-y1)-1-{[3-(trifluoroacetyl)-1H-pyrrol-1-yl]sulfonyl}urea;
3-(1,2,3,5,6,7-hexahydro-s-indacen-4-yl)-1-(1H-indole-1-sulfonyl)urea;
3-(1,2,3,5,6,7-hexahydro-s-indacen-4-yl)-1-{1H-pyrrolo[3,2-b]pyridine-1-sulfonyl}urea;
3-(1,2,3,5,6,7-hexahydro-s-indacen-4-yl)-1-(1H-indazole-1-sulfonyl)urea;
1-[(3-cyclopropyl-1H-pyrrol-1-yl)sulfonyl]-3-(1,2,3,5,6,7-hexahydro-s-indacen-4-yl)urea;
1-[(3-acetyl-1H-pyrrol-1-yl)sulfonyl]-3-(8-chloro-1,2,3,5,6,7-hexahydro-s-indacen-4-yl)urea;
3-(1,2,3,5,6,7-hexahydro-s-indacen-4-yl)-1-1H-pyrrolo[2,3-c]pyridine-1-sulfonyl}urea;
3-(1,2,3,5,6,7-hexahydro-s-indacen-4-yl)-1-[(3-nitro-1H-pyrrol-1-yl)sulfonyl]urea;
3-(1,2,3,5,6,7-hexahydro-s-indacen-4-yl)-1-[(3-propanoyl-H-pyrrol-1-yl)sulfonyl]urea;
3-{1H,2H,3H,5H,6H,7H,8H-cyclopenta[b]naphthalen-4-yl}-1-(1H-indazole-1-sulfonyl)urea;
3-{1H,2H,3H,6H,7H,8H,9H-cyclopenta[a]naphthalen-5-yl}-1-(1H-indazole-1-sulfonyl)urea;
3-(1,2,3,5,6,7-hexahydro-s-indacen-4-yl)-1-({4-oxo-2H,4H,5H,6H-cyclopenta[c]pyrrol-2-yl}sulfonyl)urea;
1-[(3-benzoyl-1H-pyrrol-1-yl)sulfonyl]-3-(1,2,3,5,6,7-hexahydro-s-indacen-4-yl)urea;
3-(1,2,3,5,6,7-hexahydio-s-indacen-4-yl)-1-{[3-(2,2,2-trifluoro-1-hydroxyethyl)-1H-pyrrol-1-yl]sulfonyl}urea;
3-(1,2,3,6,7,8-hexahydroas-indacen-4-yl)-1-{[3-(trifluoroacetyl)-1H-pyrrol-1-yl]sulfonyl}urea;
1-(6-oxo-1,2,3,6,7,8-hexahydroas-indacen-4-yl)-3-{[3-(trifluoroacetyl)-1H-pyrrol-1-yl]sulfonyl}urea;
3-(1,2,3,5,6,7-hexahydro-s-indacen-4-yl)-1-[(2-methyl-1H-pyrrol-1-yl)sulfonyl]urea;
1-[(3-acetyl-4-phenyl-1H-pyrrol-1-yl)sulfonyl]-3-(1,2,3,5,6,7-hexahydro-s-indacen-4-yl)urea; and 3-(1,2,3,5,6,7-hexahydro-s-indacen-4-yl)-1-[(4-oxo-4,5,6,7-tetrahydro-2H-isoindol-2-yl)sulfonyl]urea.

14. A compound, or pharmaceutically acceptable salt thereof, according to any one of claims 1 to 13 for use in therapy.

15. A pharmaceutical composition comprising a compound of Formula (I) according to any one of claims 1 to 13, or a pharmaceutically acceptable salt thereof and a pharmaceutically acceptable carrier or excipient.

16. A compound of Formula (I) according to any one of claims 1 to 13, or a pharmaceutically acceptable salt thereof, for use in the treatment of an autoinflammatory disorder, an autoimmune disorder, a neurodegenerative disease or cancer.

17. A compound of Formula (I) according to claim 16, or a pharmaceutically acceptable salt thereof wherein the autoinflammatory or autoimmune disorder is cryopyrin-associated autoinflammatory syndrome (CAPS) such as for example familial cold autoinflammatory syndrome (FCAS), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), gout, rheumatoid arthritis, Crohn's disease, COPD, fibrosis, obesity, type 2 diabetes, multiple sclerosis or neuro-inflammation occurring in protein miss-folding diseases, such as Prion diseases.

18. A compound of Formula (I) according to claim 16, or a pharmaceutically acceptable salt thereof wherein the neurodegenerative disease is Parkinson's disease or Alzheimer's disease.

19. A method for the treatment of an autoinflammatory disorder, an autoimmune disorder, a neurodegenerative disease or cancer in a subject in need of such treatment, said method comprising administering a therapeutically effective amount of a compound according to any of claims 1 to 13, or a pharmaceutically acceptable salt or hydrate thereof, or a pharmaceutical composition according to claim 15.

20. A method according to claim 19 wherein the autoinflammatory or autoimmune disorder is cryopyrin-associated autoinflammatory syndrome (CAPS) such as for example familial cold autoinflammatory syndrome (FCAS), Muckle-Wells syndrome (MWS), chronic infantile neurological cutaneous and articular (CINCA) syndrome, neonatal-onset multisystem inflammatory disease (NOMID), familial Mediterranean fever and nonalcoholic fatty liver disease (NAFLD), gout, rheumatoid arthritis, Crohn's disease, COPD, fibrosis, obesity, type 2 diabetes, multiple sclerosis and neuro-inflammation occurring in protein miss-folding diseases, such as Prion diseases.
